# EUROPEAN PATENT SPECIFICATION

(11) **EP 3 765 084 B1**
(45) Date of publication and mention of the grant of the patent: **15.09.2021**
(21) Application number: 19712117.1
(22) Date of filing: 05.03.2019
(51) Int. Cl.: A61K 45/06, A61K 31/785, A61K 31/787, A61K 31/79, A61P 17/02, C08F 220/20, C08F 265/06, C08L 33/24

(54) **PREPARATION BASED ON INTERPENETRATING POLYMER NETWORKS FOR WOUND HEALING**
ZUBEREITUNG AUF DER BASIS INTERPENETRIERENDER POLYMERNETZWERKE ZUR WUNDHEILUNG
PREPARATION BASEE SUR DES RESEAUX DE POLYMERES INTERPENETRES POUR LA CICATRISATION DE PLAIES

(30) Priority: 15.03.2018 CZ 201834785 U; 15.03.2018 CZ 20180133
(43) Date of publication of application: 20.01.2021
(73) Proprietor: Ustav Makromolekularni Chemie AV CR, V. V. I., 16200 Praha 6 (CZ)
(72) Inventor: POLAKOVA, Lenka, 16200 Praha 6 (CZ); SEDLAKOVA, Zdenka, 25262 Horomerice (CZ); POREBA, Rafal, 16500 Praha 6 (CZ); RYPACEK, Frantisek, 19600 Praha 9 (CZ)
(74) Representative: Hartvichova, Katerina
(86) International application number: PCT/CZ2019/050010
(87) International publication number: WO 2019/174658

(56) References cited:
- WO-A1-99/28359
- CN-A- 103 113 700
- L. POLAKOVA ET AL: "Antioxidant Properties of 2-Hydroxyethyl Methacrylate-Based Copolymers with Incorporated Sterically Hindered Amine", BIOMACROMOLECULES, vol. 16, no. 9, 14 September 2015 (2015-09-14), pages 2726-2734, XP55252383, US ISSN: 1525-7797, DOI: 10.1021/acs.biomac.5b00599
- PASLAY LEA C ET AL: "Antimicrobial poly(methacrylamide) derivatives prepared via aqueous RAFT polymerization exhibit biocidal efficiency dependent upon cation structure.", BIOMACROMOLECULES 13 AUG 2012, vol. 13, no. 8, 13 August 2012 (2012-08-13), pages 2472-2482, XP002791427, ISSN: 1526-4602

## Description

### Field of Art

Object of the invention is a preparation based on interpenetrated polymer network, the said preparation modifying the wound environment by effectively removing microorganisms as well as inflammatory processes from the wound, thereby significantly accelerating the wound healing.

### Background Art

In recent decades, there has been a significant increase in the number of patients suffering from chronic skin injuries such as leg ulcers, sores, decubitus. The ever-increasing prevalence rate in this area is significantly influenced by the growing proportion of diabetic, obese and aging individuals in the population, representing groups of patients whose immune system is usually somewhat disturbed. For example, about 2% of the population of the Czech Republic - every fiftieth person - suffers from a leg ulcer at some time during their lifetime. Statistics also show that there are currently over 650,000 patients in Germany and up to 18% of the total UK population suffering from chronic skin injuries (open leg wounds, sores, diabetic foot). It is estimated that across Europe, the current number of patients suffering from this type of chronic skin disease is around 3.5 million. All kinds of injuries significantly limit the patients, reduce the quality of their lives, and in extreme cases threaten their life.

Wound healing is a complex physiological process characterized by four different and overlapping stages (exudative/inflammatory, proliferative, differentiation, maturation/remodeling). During the first, inflammatory phase, cells of the immune system produce reactive oxygen species (ROS) that are released into the extracellular fluid, exudate. The main types of ROS include not only oxygen radicals (e.g. hydroxyl, peroxyl, hydroperoxyl radicals or superoxide anion radicals), but also oxygen compounds of an electron-neutral nature which are capable of generating reactive oxygen radicals due to their chemical decomposition (hydrogen peroxide, nitric acid, chloric acid). At low concentration, ROS play a very important role in the exudative phase of healing, contributing to wound cleansing, especially from microbial contaminants. Enzymes (proteases) are also activated in the affected cells, the activity of said enzymes includes removal of dead cells and tissues. For a wound, the healing of which does not show any abnormalities, this inflammatory phase lasts for about 2-3 days, during which the system progresses smoothly to the next stages of healing.

However, the excessive immune response may disturb the fragile balance between the rate of stimulation of pro-inflammatory mediators responsible for ROS formation and the current ROS concentration at the site of injury. As a result of this imbalance, there is a massive production of ROS, which dramatically exceeds the antioxidant capacity of the surrounding cells, thereby impairing the healing process due to increasing oxidative stress. On the cellular and molecular levels, the excess reactive radicals non-selectively attack even healthy cells, in which they are capable to peroxidize the phospholipid bilayer that is the main component of the cell membrane. This oxidative damage leads to a severe impairment of the cell outer layer integrity, followed by leakage of cytoplasm and cell death. Some radicals can penetrate into the inner cell space and irreversibly damage its DNA. Excessive local concentrations of ROS have a major impact on microorganisms present in the wound, because in oxidative stress environment, the microorganisms can create a biofilm that is highly resistant to the oxidative effect of the continuously generating ROS. This cycle of reactions practically stops the wound healing in the first exudative phase, and the wound becomes chronic.

The most common types of pathogenic microorganisms capable of persistently colonizing the environment of chronic wounds on the skin include, in particular, the strains *Staphylococcus aureus, Staphylococcus epidermidis* and *Pseudomonas aeruginosa;* mucosal infections are mainly caused by the pathogens *Escherichia coli* and *Candida albicans.* One of the reasons for the high resistance of these monocellular organisms is their ability to form a resistant biofilm under unfavorable external conditions (e.g., increased oxidative stress caused by inadequate response of the host immune system). The resistance of microbes in biofilm to current methods of treatment of microbial infections is typically one of the main causes of chronic skin and mucosal diseases.
Traditional chronic wound therapies include a wide range of disinfecting wound dressings, antibiotic ointments, tinctures, ointments and pastes containing metals. Many of these dermatological agents have a significant sensitization potential, and can thus be the cause of allergic and toxic reactions. Moreover, long-term treatment of chronic wounds by antibiotics causes the microbial resistance to antibiotics, and it is thus necessary to replace them with new, more effective treatments. Frequent use of antibiotics can cause serious systemic side effects such as damage of intestinal microflora, colitis, allergic reactions, atherosclerosis, kidney damage. Traditional wound dressings also have limited absorption capacity and do not maintain optimum humidity in the wound, which often gets dry or macerates. Also, more frequent visual wound controls and wound dressing changes are required, exposing the wound to the risk of further infection. The wound temperature decreases, and healing stagnates. The result of treatment with these traditional dressings is thus often uncertain, the healing process is lengthy, and, last but not least, uneconomical.

Based on the current knowledge of the wound healing process and on the knowledge of the effects of the hitherto known formulations, general requirements can be formulated which shall optimally be met by wound healing preparations, so that it addresses all the wound healing stages as effectively as possible.
a) In the first stage, the preparation should inhibit the development of initial inflammation, in particular have the ability to remove excess ROS, thereby limiting their toxic effects on cells and tissues, thereby preventing the chain reaction of the immune system, accompanied by the formation of further ROS.
b) Subsequently, the preparation should prevent the development of the inflammatory phase by limiting the possibility of penetration of the bacterial infection into the wound, both by mechanically covering the wound, for which it must have appropriate material and physical properties, and by actively limiting the proliferation of the bacteria by bactericidal components.
c) The preparation should support and accelerate the healing process by providing optimal physiological conditions for the actual biological healing processes, including enzymatic degradation and resorption of damaged tissue components, differentiation of new tissue cells, and gradual wound remodeling. Therefore, the dressing should have such physical properties as to avoid fluid accumulation and wound maceration, and at the same time to prevent wound drying, scab formation, and to maintain the optimal moisture level of the wound to provide a moist healing.
d) Last but not least, any biologically active ingredients optionally contained in the preparation shall not penetrate through the open wound into the internal environment of the organism and thus shall not cause undesired systemic effects. This applies both to ingredients intended for the removal of ROS and to substances with a bactericidal effect, e.g. antibiotics, whose adverse effects are discussed above.

In the literature (L. C. Paslay, et al., Biomacromolecules 2012, 13, 2472-2482), polymeric (meth)acrylamides containing covalently bound primary and tertiary amines having antibacterial properties are described. The authors also describe the mechanism of antibacterial action of these polymers, which is based on the interaction of amino groups of the polymer with the phospholipid layer of the bacterial cell, resulting in cell membrane disruption and cytolysis. The authors describe water-soluble linear polymers having the molecular weight *M*ₙ < 7 200 Da. For assessing the possibility of using such polymers to cover an open wound, it is necessary to take into account the risk that water-soluble polymers of such low molecular weight will undoubtedly penetrate into the wound and into blood circulation, and thus may exhibit undesired systemic effects outside the wound.

The preparation of hydrogel dressings for wound healing is disclosed in the patent CZ 293419 B6. This patent discloses hydrophilic classical (not interpenetrating) polymer networks in the form of a gel or film which, when applied to inflammatory skin injury, accelerate the healing process. The network-forming polymers contain a covalently bound sterically hindered amine, optionally nitroxide or hydroxylamine, which are capable of binding free radicals and acting in the wound as ROS traps (antioxidants). Polymer networks may contain, in addition to the antioxidant component, an antimicrobial component in the form of a quaternary ammonium salt which can be prepared by an additional reaction of alkyl halide with tertiary amine groups incorporated into the polymer structure. This additional quaternization brings obvious drawbacks, including the ability of alkyl halides to react with sterically hindered amines and their derivatives, thereby resulting in a reduction in the concentration of ROS-trapping active groups in the resulting polymer network. Furthermore, the polymer modification also significantly increases the cost of polymer production, both in terms of price of the starting materials and the polymer purification after the reaction, since the removal of unreacted alkylating agents from the polymer network is a time consuming and economically demanding process. Furthermore, alkyl halide compounds have a significant sensitization potential, and are also environmentally hazardous as they are toxic to the environment as well as to living organisms.
Another disadvantage of the polymer preparations according to the patent CZ 293419 B6 is the fact that the extent to which the proportion of antioxidant / antimicrobial components of the polymer network produced by a single radical polymerization can be varied is significantly limited by the mutual reactivity of the functional monomers, i.e. by their monomer reactivity ratios.

In order to prevent wound formation and accelerate wound healing, there is still a need for an improved wound dressing, especially for chronic wounds, that would perform the basic function of the barrier as well as other functions that are specific to the different stages of healing. Although a number of wound dressings are currently available in the patent literature and on the market, contributing to the acceleration of the healing process of chronic infected wounds, none of these wound dressings meets all the requirements for optimal cover material. Antimicrobial and anti-inflammatory substances are added to the hydrogel coatings as low-molecular-weight compounds or as linear polymers or as dispersed small particles that are released into the body during the application, thereby gradually reducing the effective concentration of the active ingredient at the site of action, and exposing the organism to the undesirable side-effects of these substances.

### Disclosure of the Invention

We have now found that the above-mentioned drawbacks of the prior art can be overcome by a preparation based on interpenetrating networks composed of polymers with covalently bound biologically active functional units. We have also found that the other above-mentioned drawbacks of the prior art can be removed by the use of structural units carrying a tertiary amino group and incorporated into the polymer network, thus achieving excellent antimicrobial properties of the preparation without the need for quaternization.

The present invention is defined in the appended claims. Object of the invention is a polymeric wound dressing preparation based on interpenetrating networks designed to prevent and heal wounds. The composition comprises a combination of two or more biocompatible polymer networks which are interpenetrating, i.e. they are at least partially entangled on a polymer scale but not covalently bonded to each other. In the present invention, at least one polymer network contains an antimicrobial functional component in its structure, and at least one polymer network contains in its structure a functional component capable of suppressing inflammatory processes in the wound (anti-inflammatory, antioxidant). The individual functional components are covalently incorporated into polymer networks, thereby effectively preventing their release into the body, thus preventing undesirable systemic effects. At the same time, when applied to the wound, there is no reduction in the local concentration of functional antioxidant or antimicrobial constituents due to their absorption into the body, thus significantly prolonging the time period over which they are in contact with the chronic wound, contrary to other wound healing preparations which release low-molecular-weight compounds. The approach based on interpenetrating network technology allows the introduction of antimicrobial and anti-inflammatory components into individual polymer networks, the concentration of which in the sub-networks can be controlled independently and can therefore be relatively high, which could not be achieved by, for example, incorporating all components into one polymer network, since the content of monomer units and their relative ratio is limited by the monomer reactivity ratios. Mutual interpenetration of the two polymer networks further ensures homogeneous dispersion of the functional components in the resulting polymer product, which also affects the antimicrobial and anti-inflammatory efficacy of the formulation.
The interpenetrating networks according to the present invention allow covalent incorporation of both functional components, i.e. anti-inflammatory and antimicrobial, into the structure of the individual polymer networks without having to be directly copolymerized together. In addition, the interpenetrating network technology enables the preparation of functional polymeric material from comonomers which are not copolymerizable by the same polymerization method, as different polymerization methods can be combined in the preparation of interpenetrating networks. For example, one polymer network can be prepared by radical polymerization and the other polymer network of polycondensation/polyaddition.
Polymer networks may generally be composed of polymer chains of one structural type or chains of two or more structural types, interconnected by covalent bonds in a single network. Interpenetrating networks (IPN) are systems composed of two or more polymer networks that are entangled on the macromolecular scale but are not interconnected by covalent bonds, and cannot be separated from each other without breaking the covalent chemical bonds. When the polymer networks are prepared in the form of IPN, the resulting network has to a certain extent the properties of the two or more individual networks, possibly resulting in a synergistic effect of the said properties. IPN with their properties and combinations of the properties of the entangled networks differ from conventional polymer products made up of the same structural units.

If the polymer networks are composed at least partially of hydrophilic polymers, then such networks do not dissolve in water but, in the aqueous medium, they absorb water between the chains and swell. For water-swellable polymer networks (gels), the term hydrogels is used.

The preparation according to the invention comprises at least two types of polymer networks exhibiting different functions in the healing process of chronic wounds, said polymer networks being interpenetrating. These at least two polymer networks are referred to herein as type A polymer and type B polymer.

In this text, the wording, "structural units derived from monomers" should be understood to designate structural units within the polymer chain obtained by polymerization reaction from the monomers, i.e., by cleavage of bonds in the monomers and forming of new bonds between the monomers during the polymerization reaction.

Alkyls, unless indicated otherwise, are C₁-C₈ alkyls.

The type A polymer is characterized by its ability to modify the chronic wound environment by effectively reducing the amount of pathogenic microorganisms contaminating the wound, particularly of *Staphylococcus aureus,* methicillin-resistant *Staphylococcus aureus* (MRSA), *Staphylococcus epidermidis, Escherichia coli, Pseudomonas aeruginosa, Candida albicans.*

### Type A polymer contains:

- at least one type of antimicrobial structural units A-1 derived from monomers of general formula: 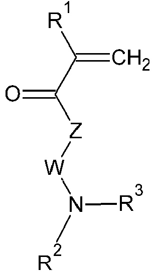 wherein R¹ is -H or -CH₃, Z is -O- or -NH-, W is -(CH₂)ₘ-, wherein m=1 to 12, R² and R³ are independently -H or C₁-C₁₂ alkyl; and
- at least one type of structural units selected from the group consisting of structural units derived from acrylamide, methacrylamide, hydroxyalkyl acrylates, hydroxyalkyl methacrylates, hydroxyalkyl acrylamides, hydroxyalkyl methacrylamides, hydroxy-terminated (polyoxyethylene) acrylates, hydroxy-terminated (polyoxyethylene) methacrylates, alkyloxy terminated (polyoxyethylene) acrylates, alkyloxy terminated (polyoxyethylene) methacrylates, hydroxy terminated (polyoxyethylene) acrylamides, hydroxy terminated (polyoxyethylene) methacrylamides, alkyloxy terminated (polyoxyethylene) acrylamides, alkyloxy terminated (polyoxyethylene) methacrylamides, wherein the polymer network containing the antimicrobial component A-1 further contains at least one type of structural units as defined in claim 1; and/or the type A polymer contains:
- at least one type of antimicrobial structural units A-2 derived from monomers of general formula: 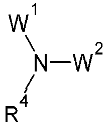 wherein R⁴ is C₁-C₁₂ alkyl, W¹ and W² are independently -(CH₂)ₙ-X¹ or -CH₂-CH₂-(OCH₂-CH₂)ₙ-X², wherein n=1 to 20, X¹ and X² are independently selected from -H, -OH, -NH₂,-NCO, wherein at least one of X¹, X² is selected from -OH, -NH₂, -NCO, wherein the polymer network containing the antimicrobial component A-2 further contains
- at least one type of structural units selected from the group consisting of
   - structural units derived from propane-1,2-diol, 2,2-dimethylpropane-1,3-diol, cyclohexane-1,4-diol, 1,4-bis(hydroxymethyl)cyclohexane, 2-(2-hydroxyethoxy)ethane-1-ol, 1,3-diaminopentane, 2,2,4-trimethylhexane-1,6-diisocyanate, 2,4,4-trimethylhexane-1,6-diisocyanate, isophorone diisocyanate, cyclohexane-1,4-diisocyanate, dicyclohexylmethane-4,4'-diisocyanate;
   - and/or structural units derived from monomers of general formula: 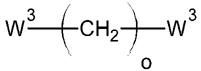 wherein o=3 to 12 and W³ is independently at each occurence -OH, -NH₂ or -NCO;
   - and/or structural units derived from monomers of general formula: 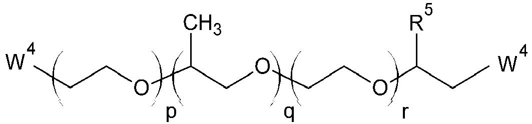 wherein p, q, r are independently an integer from 0 to 100, W⁴ is independently at each occurence -OH, -NH₂ or -NCO, and R⁵ is -H or -CH₃; wherein the polymer network containing the antimicrobial component A-2 further contains at least one type of structural units as defined in claim 1.

Salts, in particular, include alkali metal salts, unless indicated otherwise.

The structural units A-1 in the type A polymer are preferably derived from *N*-[3-(dimethylamino)propyl]methacrylamide or [2-(dimethylamino)ethyl]methacrylate.

The content of the structural units A-1 in the type A polymer is typically 1 to 99 mol%, preferably 2 to 30 mol%.

Preferably, the A-1 structural units are electroneutral or in salt form.

The structural units A-2 in the type A polymer are preferably derived from *N,N*-bis(2-hydroxyethyl)methylamine.

The content of the structural units A-2 in the type A polymer is typically 1 to 99 mol%, preferably 1 to 50 mol%.

The said type A polymer is obtainable:
a) by radical polymerization of at least one type of monomers of general formula: 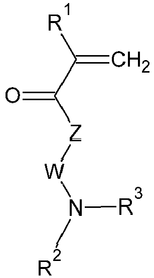 wherein R¹ is -H or -CH₃, Z is -O- or -NH-, W is -(CH₂)ₘ-, wherein m=1 to 12, R² and R³ are independently -H or C₁-C₁₂ alkyl; and preferably also
   - at least one type of monomers selected from acrylamide, methacrylamide, hydroxyalkyl acrylates, hydroxyalkyl methacrylates, hydroxyalkyl acrylamides, hydroxyalkyl methacrylamides, hydroxy-terminated (polyoxyethylene) acrylates, hydroxy-terminated (polyoxyethylene) methacrylates, alkyloxy terminated (polyoxyethylene) acrylates, alkyloxy terminated (polyoxyethylene) methacrylates, hydroxy terminated (polyoxyethylene) acrylamides, hydroxy terminated (polyoxyethylene) methacrylamides, alkyloxy terminated (polyoxyethylene) acrylamides, alkyloxy terminated (polyoxyethylene) methacrylamides,
      or
b) by polyaddition of at least one type of monomers of general formula: 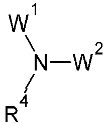 wherein R⁴ is C₁-C₁₂ alkyl, W¹ and W² are independently -(CH₂)ₙ-X¹ or -CH₂-CH₂-(OCH₂-CH₂)ₙ-X², wherein n=1 to 20, X¹ and X² are independently selected from -H, -OH, -NH₂,-NCO, wherein at least one of X¹, X² is selected from -OH, -NH₂, -NCO, and preferably also
   - at least one type of monomers selected from
      - monomers selected from propane-1,2-diol, 2,2-dimethylpropane-1,3-diol, cyclohexane-1,4-diol, 1,4-bis(hydroxymethyl)cyclohexane, 2-(2-hydroxyethoxy)ethane-1-ol, 1,3-diaminopentane, 2,2,4-trimethylhexane-1,6-diisocyanate, 2,4,4-trimethylhexane-1,6-diisocyanate, isophorone diisocyanate, cyclohexane-1,4-diisocyanate, dicyclohexylmethane-4,4'-diisocyanate;
      - and/or monomers of general formula: 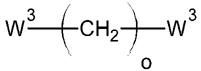 wherein o=3 to 12 and W³ is independently at each occurence -OH, -NH₂ or -NCO;
      - and/or monomers of general formula: 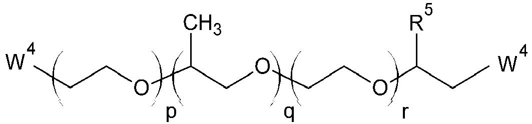
   wherein p, q, r are independently an integer from 0 to 100, W⁴ is independently at each occurence -OH, -NH₂ or -NCO, and R⁵ is -H or -CH₃.

Type B polymer is characterized by its ability to affect the wound enviroment due to its content of chemical moieties capable of binding or inactivating of compounds undesirably influencing the inflammatory processes in the wound, i.e., antioxidant and/or antiinflammatory moieties.

### The said type B polymer contains:

- at least one type of antiinflammatory and/or antioxidant structural units B-1 derived from monomers of general formula: 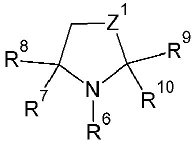 wherein R⁶ is selected from -H, -OH, oxygen radical and C₁-C₄ alkyl, R⁷ to R¹⁰ are independently C₁-C₄ alkyl, Z¹ is -CH(Y¹)- or -CH(Y¹)CH₂-, wherein Y¹ is radical-polymerizable chemical moiety 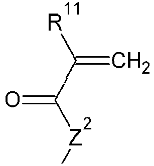 wherein R¹¹ is -H or -CH₃ and Z² is -O- or -NH-; and
- at least one type of structural units selected from the group consisting of structural units derived from acrylamide, methacrylamide, hydroxyalkyl acrylates, hydroxyalkyl methacrylates, hydroxyalkyl acrylamides, hydroxyalkyl methacrylamides, hydroxy-terminated (polyoxyethylene) acrylates, hydroxy-terminated (polyoxyethylene) methacrylates, alkyloxy terminated (polyoxyethylene) acrylates, alkyloxy terminated (polyoxyethylene) methacrylates, hydroxy terminated (polyoxyethylene) acrylamides, hydroxy terminated (polyoxyethylene) methacrylamides, alkyloxy terminated (polyoxyethylene) acrylamides, alkyloxy terminated (polyoxyethylene) methacrylamides; and wherein the polymer network containing the antiinflammatory and/or antioxidant component B-1 further contains at least one type of structural units as defined in claim 1.
   and/or the type B polymer contains:
- at least one type of antiinflammatory and/or antioxidant structural units B-2 derived from monomers of general formula: 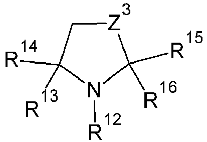 wherein R¹² is selected from -H, -OH, oxygen radical and C₁-C₄ alkyl, R¹³ to R¹⁶ are independently C₁-C₄ alkyl, Z³ is -CH(Y²)- or -CH(Y²)CH₂-, wherein Y² is -OH, -NH₂,-NHC(=O)NH₂, -(CH₂)ₛ-W⁵, wherein s=1 to 6 and W⁵ is -OH or -NH₂;
   and at least one type of structural units selected from the group comprising
   - structural units derived from propane-1,2-diol, 2,2-dimethylpropane-1,3-diol, cyclohexane-1,4-diol, 1,4-bis(hydroxymethyl)cyclohexane, 2-(2-hydroxyethoxy)ethane-1-ol, 1,3-diaminopentane, 2,2,4-trimethylhexane-1,6-diisocyanate, 2,4,4-trimethylhexane-1,6-diisocyanate, isophorone diisocyanate, cyclohexane-1,4-diisocyanate, dicyclohexylmethane-4,4'-diisocyanate;
   - and/or structural units derived from monomers of general formula: 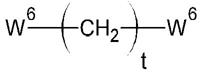 wherein t=3 to 12, and W⁶ is independently at each occurence -OH, -NH₂ or -NCO;
   - and/or structural units derived from monomers of general formula: 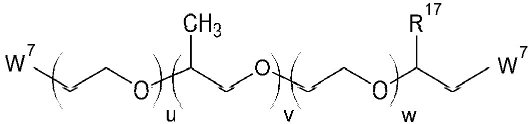 wherein u, v, w are independently an integer from 0 to 100, R¹⁷ is -H or -CH₃ and W⁷ is independently at each occurence -OH, -NH₂ or -NCO; wherein the polymer network containing the antimicrobial component B-2 further contains at least one type of structural units as defined in claim 1.

The structural units B-1 in the type B polymer are preferably derived from *N*-(2,2,6,6-tetramethylazinan-4-yl) methacrylamide or 2,2,6,6-tetramethylazinan-4-yl methacrylate.

The content of B-1 structural units in the type B polymer is typically 1 to 45 mol%, preferably 1.5 to 15 mol%.

The structural units B-2 in the type B polymer are preferably derived from 4-amino-2,2,6,6-tetramethylpiperidine, 4-amino-2,2,6,6-tetramethylpiperidine-1-oxyl, 4-hydroxy-2,2,6,6-tetramethylpiperidine or 4-hydroxy-2,2,6,6-tetramethyl-piperidine-1-oxyl.

The content of the structural units B-2 in the type B polymer is typically 0.1 to 20 % by weight.

The said type B polymer is obtainable:
a) by radical polymerization of at least one type of monomers of general formula: 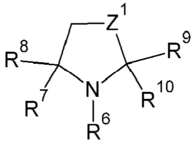 wherein R⁶ is selected from -H, -OH, oxygen radical and C₁-C₄ alkyl, R⁷ to R¹⁰ are independently C₁-C₄ alkyl, Z¹ is -CH(Y¹)- or -CH(Y¹)CH₂-, wherein Y¹ is radical-polymerizable chemical moiety 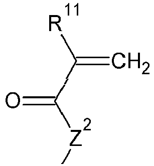 wherein R¹¹ is -H or -CH₃ and Z² is -O- or -NH-; and
   - at least one type of monomers selected from acrylamide, methacrylamide, alkyl acrylates, hydroxyalkyl methacrylates, hydroxyalkyl acrylamides, hydroxyalkyl methacrylamides, hydroxy-terminated (polyoxyethylene) acrylates, hydroxy-terminated (polyoxyethylene) methacrylates, alkyloxy terminated (polyoxyethylene) acrylates, alkyloxy terminated (polyoxyethylene) methacrylates, hydroxy terminated (polyoxyethylene) acrylamides, hydroxy terminated (polyoxyethylene) methacrylamides, alkyloxy terminated (polyoxyethylene) acrylamides, alkyloxy terminated (polyoxyethylene) methacrylamides;
      or
b) by polyaddition of at least one type of monomers of general formula: 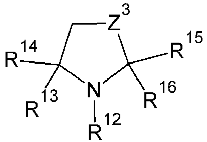 wherein R¹² is selected from -H, -OH, oxygen radical and C₁-C₄ alkyl, R¹³ to R¹⁶ are independently C₁-C₄ alkyl, Z³ is -CH(Y²)- or -CH(Y²)CH₂-, wherein Y² is -OH, -NH₂,-NHC(=O)NH₂, -(CH₂)ₛW⁵, wherein s=1 to 6 and W⁵ is -OH or -NH₂;
   and preferably at least one type of monomers selected from the group comprising
   - monomers selected from propane-1,2-diol, 2,2-dimethylpropane-1,3-diol, cyclohexane-1,4-diol, 1,4-bis(hydroxymethyl)cyclohexane, 2-(2-hydroxyethoxy)ethane-1-ol, 1,3-diaminopentane, 2,2,4-trimethylhexane-1,6-diisocyanate, 2,4,4-trimethylhexane-1,6-diisocyanate, isophorone diisocyanate, cyclohexane-1,4-diisocyanate, dicyclohexylmethane-4,4'-diisocyanate;
   - and/or monomers of general formula: 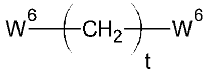 wherein t=3 to 12, and W⁶ is independently at each occurence -OH, -NH₂ or -NCO;
   - and/or monomers of general formula: 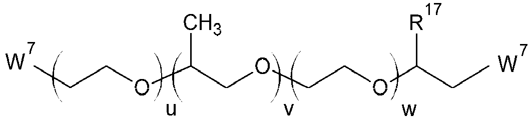 wherein u, v, w are independently an integer from 0 to 100, R¹⁷ is -H or -CH₃ and W⁷ is independently at each occurence -OH, -NH₂ or -NCO.

The weight ratio of type A polymers to type B polymers in the preparation of the present invention is in the range of 0.1 to 10, wherein an appropriate adjustment of this ratio within the said interval allows to optimize the range of properties that are required from the resulting polymeric wound healing preparation.

Type A polymers and type B polymers are in the form of interpenetrating networks, that is, they are not interconnected by covalent bonds but are entagled so that they cannot be separated without breaking covalent bonds in the polymers. Typically, the interpenetrating networks are obtained by first forming a first polymeric network from first monomers, the first polymeric network is subsequently impregnated with second monomers, and the second monomers are then polymerized to form a second polymer network. This procedure can be repeated if it is desired to create more interpenetrating networks.

Interpenetrating networks (IPN) thus represent a practical possibility of preparation or production of a product with optimal physico-chemical properties, which cannot be normally achieved by a simple copolymerization of different functional monomers into a single polymer chain, or by a simple mixing of copolymers, copolymer and terpolymer networks, block copolymers or graft polymers.
The advantage of the IPN technology is that in an existing polymer network prepared by a single polymerization process, synthesis of the second or additional polymer network through a different polymerization process using another polymerization mechanism is possible. For example, in a polymer network formed by radical polymerization of monomers, a second polymer network can be prepared by polymerization of monomers by polyaddition or polycondensation. This allows to produce a resulting IPN containing structural units derived from different monomers which otherwise would not, due to their chemical structure and reactivity, form a polymer together.

According to the present invention, the content of functional groups active in the binding of ROS (antioxidant, anti-inflammatory) may be adjusted independently on the content of antimicrobial structural units, within a wide range of concentrations thereof, both moieties being covalently bound to the polymer network, thus they cannot be absorbed into the bloodstream and cannot cause undesirable systemic side-effects. Thus, the functional groups are exclusively localized on the surface of the wound.

Antimicrobial or bactericidal action of the polymeric preparation of the invention can be achieved by incorporating structural units containing tertiary amines or quaternary ammonium salts. These structures are ionogenic and charged at physiological pH. The presence of ionogenic groups in a simple polymeric network increases its swelling in aqueous environment and thus can negatively influence its mechanical properties. According to the present invention, this effect of the ionogenic groups in the resulting preparation can be compensated by the choice of interpenetrating networks structure and by the presence of a further network containing structural units favoring good mechanical properties such as strength, elasticity and resilience. This allows to achieve favorable mechanical properties of the product even with a higher content of ionogenic bactericidal components. The polymeric preparation of the present invention for wound prevention and wound healing can preferably be applied to the affected site in the form of a dressing sheet, suspension, emulsion, gel or ointment. The protective wound dressing is elastic, breathable, or even transparent, allowing continuous visual inspection of the wound.

The preparation is chemically stable in the microenvironment of the wound, and does not undergo chemical degradation in the presence of wound secretion. In contact with the exudate, the preparation swells and thus removes the exudate from the wound. The interpenetrating polymer networks which form the basis of the preparation contain within their structure functional components that are capable of binding the contaminants in the exudate, thus positively contributing to the healing process.

To initiate the radical copolymerization, initiators are used so that they correspond to the selected polymerization process and the resulting polymerization conditions. The most common types of radical initiators include, for example, thermal initiators (azo-initiators, diacyl peroxides and other types of peroxo compounds), UV initiators that generate radicals by UV irradiation, or redox initiators that generate radicals by oxidation-reduction reactions. However, other initiators can be used.

For the preparation of crosslinked polymers by radical copolymerization, crosslinking monomers selected from the group consisting of ethylene glycol di(meth)acrylate, oligo (ethylene glycol) di(meth)acrylates, *N,N'*-methylene bis(meth)acrylamide, *N,N'*-ethylenebis(meth)acrylamide, 1'-divinyl-3,3'-(ethane-1,1'-diyl)bis(2-pyrrolidinone), 2,3-dihydroxybutane-1,4-diyl-di(meth)acrylate, or other crosslinking agents known in this field may be used.

Catalysts can be used to accelerate the polyaddition reaction at laboratory and industrial scale, taking into account the composition of the initial polymerization mixture and the structure of the resulting polymer. Suitable types of catalysts include organic bases, organic acids and organometallic compounds, including 1,4-diazabicyclo[2.2.2]octane, 1,8-diazabicyclo[5.4.0]undec-7-ene, 1,5,7-triaza-bicyclo[4.4.0]dec-5-ene, *N,N,N'*,*N'*-tetramethyl-2,2'-oxybis(ethylamine), *N,N*-dimethyl-*N-*hexadecylamine, *N,N*-dimethyl-*N*-cyclohexylamine, *N,N*-dimethyl-*N*-benzylamine, *N,N*-bis(2-dimethylaminoethyl)-*N*-methylamine, *N,N*-dicyclohexyl-*N*-methylamine, diphenylphosphate, methanesulfonic acid, trifluoromethanesulfonic acid, iron(III) acetylacetonate, dibutyltin dilaurate, dibutyltin dichloride, triethylenediamine. However, other catalysts may be used. The use of various types of catalysts known to those skilled in the art of polyaddition reactions is in accordance with the present invention.

For the preparation of crosslinked polymers by polyaddition, a compound selected from polyfunctional alcohols or polyfunctional amines may be used as a crosslinker, including for example low-molecular-weight propane-1,2,3-triol, 2,2-bis(hydroxymethyl)propane-1,3-diol, bis(2-hydroxyethyl)amine, tris(2-hydroxyethyl)amine, 2-(hydroxymethyl)-2-ethylpropane-1,3-diol, or other crosslinkers commonly used in the art. Preferably, the crosslinkers may also be -OH, -NH₂ or -NCO-terminated polymeric precursors based on block and star-like copolymers of ethylene glycol and propylene glycol having a functionality greater than 2.

The term "oligo" refers to 2 to 20 repeating structural units.

In addition to the interpenetrating polymer network, the preparation of the present invention may contain further pharmaceutically acceptable excipients, depending on the final form of the composition, which according to the particular application may be a liquid form (suspension, emulsion), a semi-solid form (ointment, cream, gel, paste), a solid unit dose preparation (suppositories, globules, tablets), wound dressing film (for application in dry or swollen form), a component of multi-layer coating. Such excipients include, for example, stabilizers, emulsifiers or viscosity enhancers, as well as paraffins, vegetable oils, animal fats, synthetic acylglycerols, waxes, polyalkylsiloxanes, colloidal silicon dioxide with fatty oils, starch, cellulose derivatives, carbomers, magnesium-aluminium silicates, gelatine, surfactants, lubricants, substances with adsorbent properties, water, glycerol. Types of excipients suitable for the respective dosage forms are well known in the pharmaceutical formulation art. In addition, the composition may contain a filler for absorbing the odor from the chronic wound, preferably activated charcoal.

The IPN of the present invention may be embodied in various dosage forms.
One of the suitable forms is IPN particles which swell in a suitable medium to form a soft microgel suspension. Suitable media for the formation of such suspensions are water, aqueous salt solutions and water-soluble or water-miscible physiologically compatible substances. Depending on the content and the swellability of the IPN particles, the consistency of the suspension can vary in a wide range from free-flowing fluids applicable e.g. by spraying, through spreadable gels, to semi-solid pastes. The particle size of IPN in these suspensions can be in the range from micrometers to several millimeters. The IPN particles for these purposes can be prepared already during the network polymerization process, e.g., using dispersion or precipitation polymerization processes, or may be obtained by mechanical disintegration of dried gel by grinding or milling.
Another suitable IPN application form are flat polymeric dressings, e.g. in the form of films or foils. The films may be prepared by thin-film polymerization, using e.g. casting, or by polymerization in a form having suitable geometry. Polymer networks forming the IPN in the film may be bi-continuous or one of the networks may form discrete domains in another continuous network. The IPN film formed by bi-continuous networks can, for example, be prepared by swelling of a type A polymer in a polymerization mixture for type B polymer and by subsequent polymerization of the mixture. The IPN film containing the discrete domains of one of the networks may, for example, be formed by dispersing and swelling of type A polymer network particles in a polymerization mixture for type B polymer and by subsequent polymerization of the mixture into a suitable form.
Various alternative techniques for pharmaceutical form manufacturing are known in the art for the preparation of wound dressings, and their use is in accordance with the present invention.

The preparations of the invention may further comprise other pharmaceutically active ingredients, for example, components intended for local release from the polymeric matrix. Such components include analgesics, wound healing accelerators, growth factors, vitamins (especially C and E), cosmetics, etc. Such components may be introduced into the polymeric matrix formed by an IPN of at least one type A polymer and at least one type B polymer by known methods, for example by impregnation or adsorption, and may be bound in the matrix by non-covalent interactions or by pH- or moisture-dependent bonds, which facilitate their desirable local release at the application site.

The industrial applicability of the present invention results from the key features of the product. Antimicrobial functional groups killing bacteria or protecting the wound against bacterial infection, and anti-inflammatory functional groups inhibiting the development of inflammatory processes in the wound are covalently incorporated into the polymeric material of the preparation, effectively preventing their release into the bloodstream and possible undesirable systemic effects.

The interpenetrating network in the form of at least two polymers specified in the description of the invention is not absorbed by the skin or mucous membrane, does not enter the bloodstream nor decompose into low-molecular-weight substances that could be absorbed by the body. The polymers are chemically neutral and are not subject to degradation in the wound environment.

The structure of the interpenetrating networks allows the polymeric formulation to exhibit high swellability in the exudate of chronic wounds. Upon application of the preparation on the wound, the wound drying is prevented, and simultaneously, the biologically active structural units embedded in the IPN structure remain in direct contact with factors affecting healing, especially ROS and infecting microorganisms.

### Brief Description of Drawings:

Fig. 1 Time dependence of the fluorescein fluorescence relative intensity in the presence of *in situ* generated peroxyl radicals and the different IPN concentrations prepared according to Example 1 (3 1 mg, ○ 2 mg, ● 5 mg; ■ blind sample).
Fig. 2 Photodocumentation of the antimicrobial effect of IPN according to Example 4 against *Staphylococcus aureus* and *Staphylococcus epidermidis* (A, C). Polyurethane matrix was chosen as reference (B, D).
Fig. 3 Viability of human fibroblasts after 24 h incubation at 37 °C with extract of the IPN of Example 4 at different dilutions.
Fig. 4 Scavenging activity of the IPN in the form of the film of Example 7 against *in situ* generated superoxide anion-radicals, depending on the concentration of the sample in the reaction mixture.
Fig. 5 Scavenging activity of IPN, terpolymer and mixtures of two networks against *in situ* generated peroxyl radicals after 60 min of reaction depending on the concentration of samples in the reaction mixture.
Fig. 6 Scavenging activity of IPN, terpolymer and mixtures of two networks against hydrogen peroxide depending on the concentration of samples in the reaction mixture.

### Examples

### Example 1

A mixture of 16.0 g of 2-hydroxyethyl methacrylate, 1.0 g of *N*-(2,2,6,6-tetramethylazinan-4-yl) methacrylamide, 120 mg of ethylene glycol dimethacrylate and 100 mg of 2,2'-azobis(2-methylpropionitrile) was dissolved in 200 mL of toluene. The polymerization reaction was carried out for 24 hours at 70 °C. The resulting polymer in the form of polymer network particles was then filtered off, repeatedly extracted with hot toluene and vacuum-dried. 10 g of the polymer network was swollen with a mixture of 45 g of toluene, 1.2 g of *N*-[3-(dimethylamino)propyl] methacrylamide, 3.8 g of 2-hydroxyethyl methacrylate, 54 mg of ethylene glycol dimethacrylate and 148 mg of 2,2'-azobis(2-methyl-propionitrile). The polymerization was carried out for 24 hours at 70 °C. The resulting IPN was filtered off, washed with toluene:acetone (2:1) at 50 °C and dried under vacuum.

### Example 2

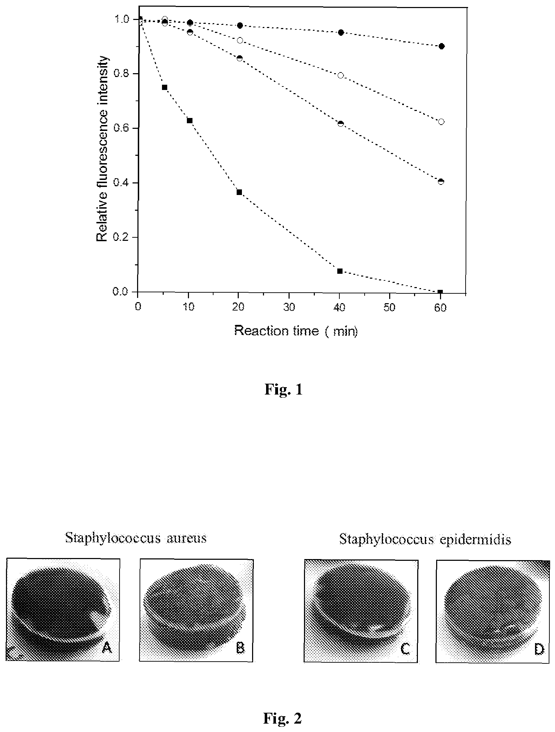

The antioxidant activity of the IPN of Example 1 was tested using peroxyl radicals. Generation of these reactive oxygen radicals proceeded by thermal decomposition of 2,2'-azobis(2-methyl-propionamidine) dihydrochloride (AAPH), wherein the *in situ* formed peroxyl radicals oxidize fluorescein, resulting in a decrease in the intensity of its fluorescence in the measured solution. Therefore, the presence of peroxyl radicals and antioxidant can be detected fluorimetrically. The experiment was carried out according to the following procedure. Into six test tubes containing the tested polymer, 1.4 mL of phosphate buffer (PBS, 20 mM, pH = 6.8) and 300 µL fluorescein solution in PBS (2.4 x 10⁻⁵ M) were added. The samples were tempered in a thermoblock for 60 min at 37 °C. Generation of peroxyl radicals was started by the addition of 100 µL of 2,2'-azobis(2-methyl-propionamidine) dihydrochloride solution in PBS (0.46 M). At pre-determined time intervals, after filtering off the polymer, the intensity of the fluorescence of the filtrate was measured. For each sample, the measurement was repeated twice. The results show (Figure 1) that the IPN exhibits high, concentration-dependent efficiency against the *in situ* generated peroxyl radicals.

### Example 3

The antimicrobial activity of the IPN prepared according to Example 1 was tested as follows. 10.0 g IPN was swollen in a mixture of 55 mL of poly(ethylene glycol) (average *M*ₙ = 300) and 45 mL of water to form a gel. Microbial strains of *Staphylococcus aureus* MRSA, *Pseudomonas aeruginosa* and *Candida albicans* were sterile inoculated directly into the gel, and using the method of counting colony forming units (cfu) on solid soils at pre-determined time intervals, the efficacy of the gel against individual strains was determined. From the results shown in Table 1 it is clear that the IPN showed high efficacy against said microbial strains.

**Table 1 Antimicrobial activity of the gel containing the IPN of Example 1.**

| Inoculated microorganism | Applied dose cfu/1 mL | Number of colony forming units in 1 mL (cfu/ 1 mL) | | | | |
|---|---|---|---|---|---|---|
| | day 0 | 6 hours | 12 hours | 24 hours | 36 hours | 48 hours |
| *S. aureus* MRSA | 4.6 x 10⁶ | 5.5 x 10⁴ | 5.6 x 10³ | 1.1 x 103 | 3 x 10² | <1 x 10¹ |
| *P. aeruginosa* CCM 1961 | 2.5 x 10⁶ | <1 x 10¹ | <1 x 10¹ | <1 x 10¹ | <1 x 10¹ | <1 x 10¹ |
| *C. albicans* CCM 8215 | 5.6 x 10⁵ | 7.6 x 10³ | 8.0 x 10² | <1 x 10¹ | <1 x 10¹ | <1 x 10¹ |

### Example 4

A mixture of 1.3 g of [2-(dimethylamino)ethyl]methacrylate, 0.9 g of poly(ethylene glycol)-methyl ether methacrylate (average *M*ₙ = 500), 54 mg of ethylene glycol dimethacrylate and 120 mg of 2,2'-azobis(2-methyl-propionitrile) was dissolved in 29 mL of toluene. The polymerization was carried out for 24 hours at 70 °C. After completion of the reaction, the resulting polymer (type A polymer) was isolated by filtration, washed with hot toluene and dried under vacuum. 25.0 g of type A polymer network was homogeneously dispersed in a mixture of 150 g of poly(ethylene glycol) (*M*ₙ = 600) and 50 g of acetone. Subsequently, 0.9 g of propane-1,2,3-triol, 9.0 g of *N,N,N',N'-*tetramethyl-2,2'-oxybis(ethylamine) and 56.8 g of *n*-hexane-1,6-diisocyanate in 200 g of acetone were added. The mixture was purged with nitrogen and allowed to react for 1 hour at 50 °C. Solution of 30.0 g of 2,2,6,6-tetramethyl-4-aminopiperidine in acetone was then added to the prepolymer and the reaction continued for a further 2 hours at 50 °C. The thus prepared mixture was poured onto a Teflon pad; after complete evaporation of the solvent, a homogeneous, transparent, flexible film formed of the IPN of type A polymer in the polyurethane network (type B polymer) was obtained.

### Example 5

The IPN of Example 4 was tested for antimicrobial activity against *Staphylococcus aureus* and *Staphylococcus epidermidis* strains. Bacterial cultures were sterile dispersed in liquid medium, and 100 µL of this suspension was uniformly dispersed on a 2 cm diameter blood agar wheel. The wheel was immediately covered by the tested film. For comparison, the polyurethane matrix (type B) alone, which does not contain the type A polymer network with an antimicrobial component, was tested in the same manner. After incubation at 37 °C for 24 hours, the growth of bacterial colonies was photographed. It is clear from the results that while the bacterial cultures grow under the film made of type B polymer matrix alone, without type A polymer matrix (Figure 2B, D), the IPN cover of Example 4 inhibits bacterial colonization (Figures 2A, C).

### Example 6

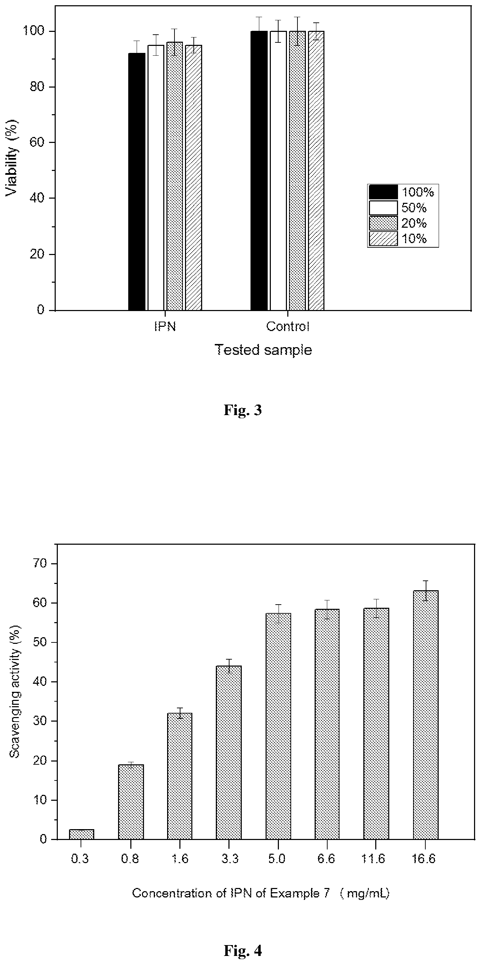

The cytotoxicity of the IPN of Example 4 in the form of the film was determined on a human fibroblast cell line using an assay according to ISO 10993-5:2009 (*In vitro* cytotoxicity assay). Cell viability was evaluated after 24-hour incubation with extracts of both samples at 37 °C, using the Alamar Blue™ Cytotoxicity Assay, which is a standard procedure recommended by the EURL-ECVAM database (The European Union Reference Laboratory for alternatives to animal testing). The mean fluorescence of negative control representing 100% viability was used to calculate cell culture viability after incubation with the test sample. The experiment was repeated three times. The results are summarized in Fig. 3. It is clear from the results that IPN extracts are nontoxic for human fibroblast cells at the concentrations given by the ISO standard.

### Example 7

A mixture of 1.0 g of 1-methyl-2,2,6,6-tetramethylazinan-4-yl methacrylate, 10.5 g of 2-methoxyethyl methacrylate, 64 mg of ethylene glycol dimethacrylate and 151 mg of 2,2'-azobis(2-methylpropionitrile) was dissolved in 72 mL of toluene. The polymerization was carried out for 24 hours at 70 °C. After completion of the reaction, the polymer was isolated by filtration, repeatedly washed with toluene and dried. 43.0 g of prepared polymer network and 106 g of poly(ethylene glycol) (*M*ₙ = 600) were stirred for 2 hours at room temperature. 9.9 g of *N*-methyldiethanolamine, 0.9 g of propane-1,2,3-triol, 42.0 of poly(propylene glycol) *α,ω*-terminated by tolylene diisocyanate (*M*ₙ = 2 300) and 60.0 g of *n*-hexane-1,6-diisocyanate. The mixture was evacuated to remove air bubbles. After being heated to 40 °C, it was poured into several Teflon molds. The curing reactions were carried out in an inert atmosphere for 8 hours at 60 °C. At the end of the reaction, transparent flexible films of different thickness were obtained.

### Example 8

IPN in the form of a film prepared according to Example 7 was tested for antioxidant activity against *in situ* generated superoxide anion radicals. A pre-determined amount of the film was weighed into 5 mL vials; 800 µL of Tris-HCl buffer, 200 µL of hypoxanthine solution and 2 mL of tetrazolium nitroblue solution were added. The reaction was started by the addition of 20 µL xanthine oxidase enzyme suspension. The reaction mixtures were stirred for 20 minutes at room temperature. The absorbance value of the solutions at 572 nm was measured. All sample concentrations were measured three times to ensure reproducibility of the experiment. From the results, it is clear (Figure 4) that under the given conditions, IPN shows already at relatively low concentrations high levels of scavenging activity against *in situ* generated superoxide anion radicals.

### Example 9

A mixture of 150 g of poly (ethylene glycol) (*M*ₙ = 600), 0.9 g of propane-1,2,3-triol, 9.0 g of *N,N,N* ',*N'*-tetramethyl-2,2'-oxybis (ethylamine), 56.8 g of *n*-hexane-1,6-diisocyanate and 250 g of acetone was bubbled through nitrogen and allowed to react for 1 hour at 50 ° C. 30.0 g of a 1-ethyl-2,2,6,6-tetramethyl-4-aminopiperidine solution in acetone was then added to the prepolymer and the reaction continued for a further 2 hours at 50 °C. The mixture thus prepared was poured onto a Teflon mould; after complete evaporation of the solvent, a transparent, flexible film was formed. 10.0 g of this film was then swollen homogeneously in an inert atmosphere in a solution of [2- (dimethylamino) ethyl] methacrylate, poly (ethylene glycol) methacrylate (average *M*ₙ = 360), 2-hydroxypropyl methacrylamide, ethylene glycol dimethacrylate and 4,4'-azobis (4-cyanovaleric) in water. The polymerization was carried out by UV irradiation of the reaction mixture at 350 nm for 30 minutes. The resulting IPN was then thoroughly washed with water to remove unreacted components and soluble oligomeric moieties. After drying, a transparent, flexible film was obtained.

### Example 10

A mixture of 5.8 g of 2,2,6,6-tetramethylazinan-4-yl methacrylate, 1.8 g of 2-hydroxyethyl methacrylamide and 144 mg of ethylene glycol dimethacrylate was dissolved in 45 mL of cyclohexane. After addition of 112 mg of 2,2'-azobis(2-methylpropionitrile), the mixture was heated at 60 °C for 18 hours. The resulting polymer network was then filtered off, repeatedly extracted with hot cyclohexane, and dried under vacuum. 8.0 g of the prepared polymer network was swollen in 45 mL of ethanol, 15 mL of heptane, 1.2 g of [2-(dimethylamino)ethyl]methacrylate, 10.0 g of poly(ethylene glycol)methacrylate (average *M*ₙ = 360), 13.6 g of 2-hydroxyethyl methacrylate, 327 mg of ethylene glycol dimethacrylate and 0.41 g of 2,2'-azobis(2-methylpropionitrile). The polymerization was carried out for 24 hours at 70 °C. Afetr filtration, the resulting IPN was repeatedly washed with hot heptane. After drying, it was subsequently extracted with distilled water at room temperature, filtered off, and lyophilized. 10.0 g of this IPN having a high content of antioxidant component was swollen in a mixture of 55 mL of poly(ethylene glycol) (average *M*ₙ = 300) and 45 mL of water. The resulting hydrogel was used to treat a decubitus on the heel which was contaminated with a yellow-green biofilm (predominantly *Pseudomonas aeruginosa*). After 24 days of treatment, the infection was eliminated and new granulation tissue, growing over the wound, was formed.

### Example 11

A mixture of 1.6 g of 2-hydroxypropyl methacrylamide, 10.2 g of *N*-[3-(dimethylamino)propyl]methacrylamide, 120 mg of *N,N'*-methylene bisacrylamide and 100 mg of 2,2'-azobis(2-methyl-propionitrile) was dissolved in 200 mL of toluene. The polymerization reaction was carried out for 24 hours at 70 °C. The resulting polymer was then filtered off, repeatedly extracted with hot toluene and dried under vacuum. 10.0 g of the polymer network was swollen in a mixture of 45 g of heptane, 1.5 g of *N*-(2,2,6,6-tetramethylazinan-4-yl) methacrylate, 8.6 g of 2-hydroxyethyl methacrylate, 54 mg of ethylene glycol dimethacrylate and 148 mg of 2,2'-azobis(2-methylpropionitrile). The polymerization was carried out for 24 hours at 70 °C. The resulting IPN with high content of antimicrobial component was filtered off while hot, washed with heptane at 50 °C and dried under vacuum. The IPN was dispersed in an ointment base containing 20 % w/w water, Macrogol 300 and cetyl palmitate. The resulting cream was applied to the external area of genital affected by yeast infection (*Candida albicans*)*.* Reduction in inflammation was evident after two applications.

### Example 12

A mixture of 10.0 g of 1-methyl-2,2,6,6-tetramethylazinan-4-yl methacrylate, 2.5 g of 2-methoxyethyl methacrylate, 48 mg of ethylene glycol dimethacrylate and 151 mg of 2,2'-azobis(2-methylpropionitrile) was dissolved in 53 mL of toluene. The polymerization was carried out for 24 hours at 70 °C. After completion of the reaction, the polymer was isolated by filtration, repeatedly washed with toluene and dried. 5.0 g of the prepared polymer network was swollen with a mixture of 45 mL of ethanol, 5.2 g of [2-(dimethylamino)ethyl]acrylate, 1.0 g of 4-hydroxybutyl acrylate, 19 mg of ethylene glycol diacrylate and 85 mg of 2,2-azobis(2-methyl-propionitrile). The polymerization was carried out for 24 hours at 70 °C. The resulting IPN with a high content of antioxidant and antimicrobial components was filtered off and washed with hot ethanol. The IPN was neutralized with dilute hydrochloric acid and subsequently suspended in water. This suspension was repeatedly applied to skin inflammation on feet, toes and fingers, which was predominantly colonized by *Candida albicans.* After three to four applications a significant improvement in the condition was observed.

### Example 13

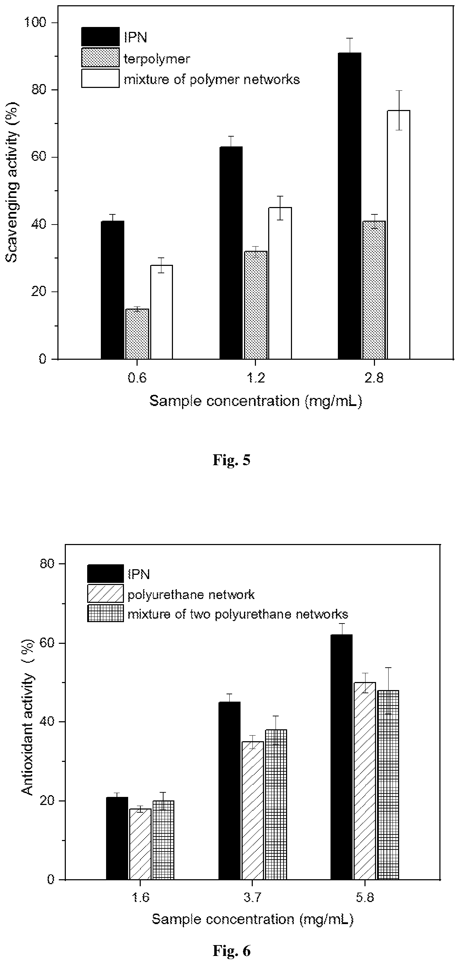

Antioxidant activity against peroxyl radicals of the IPN sample of Example 1 was compared with the antioxidant activity of other types of polymer networks having the same antioxidant and antimicrobial components content, namely a sample in the form of a simple terpolymer network (containing the antioxidant and antimicrobial component in a single network), and a sample formed by mixing a copolymer network containing the antioxidant with a copolymer network containing the antimicrobial component. The samples were tested for antioxidant activity against *in situ* generated peroxyl radicals. The experiment was carried out according to the procedure described in Example 2. The results of the experiment are summarized in Fig. 5, which represents the dependence of the scavenging activity of the test samples on its concentration in the reaction mixture containing the peroxyl radicals. The results show that the IPN exhibits a higher scavenging activity against *in situ* generated peroxyl radicals than both the terpolymer network and the mixture of two copolymer networks.

### Example 14

Antimicrobial activity of the IPN sample of Example 1 was also compared with the antimicrobial activity of the terpolymer network and of the mixture of two polymer networks, all samples having the same content of the incorporated antioxidant and antimicrobial components. The polymers were swollen with a mixture of poly(ethylene glycol) (average *M*ₙ = 300) and 45 mL of water to form gels. The antimicrobial activity of the prepared hydrogels was tested against the strain of Gram-positive bacteria *Staphylococcus aureus* MRSA, Gram-negative bacteria *Pseudomonas aeruginosa,* and yeast *Candida albicans.* To 10 mL of the gel, 0.1 mL of inoculum containing a pre-determined number of cfu was inoculated under sterile conditions. The inoculated preparation was stored at 32 °C (for bacteria) or at 22 °C (for yeast) for 12 hours. The antimicrobial effect (in %) is shown in Table 2 for the individual gels. The results show that IPN has the highest efficacy against the tested microbial cultures.

**Table 2 Antimicrobial effect of tested hydrogels against Staphylococcus aureus MRSA, Pseudomonas aeruginosa and Candida albicans after 12 hours of incubation.**

| Polymer network type | Microbial strain | Antimicrobial effect (%) |
|---|---|---|
| terpolymer | *Staphylococcus aureus* (MRSA) | 94.8 |
| mixture of networks | | 96.1 |
| IPN | | 99.9 |
| terpolymer | *Pseudomonas aeruginosa* (CCM 1961) | 96.4 |
| mixture of networks | | 95.3 |
| IPN | | 99.9 |
| terpolymer | *Candida albicans* (CCM 8215) | 91.1 |
| mixture of networks | | 94.6 |
| IPN | | 99.9 |

### Example 15

A mixture of 50.0 grams of poly(ethylene glycol) *α*, *ω*-terminated by primary amine (*M*ₙ = 2 000), 2.0 grams of *N*-ethyldiethanolamine and 1.0 g of propane-1,2,3-triol was dissolved in 75 mL of acetone, then 9.6 g of 2,4,4-trimethylhexane-1,6-diisocyanate was added to the solution, the reaction mixture was purged with nitrogen and allowed to react for 3.5 hours at 50 °C. The mixture was then poured onto a Teflon mould, and after evaporation of the solvent a thin transparent film was obtained. 15.0 g of this film was swollen on the mould for 5 hours in a solution of 30.0 g of poly(ethylene glycol) (*M*ₙ = 600) and 35 mL of acetone. Then, 0.4 g of propane-1,2,3-triol, 3.5 g of *N,N,N',N'*-tetramethyl-2,2'-oxybis(ethylamine) and 8.5 g of *n*-hexane-1,6-diisocyanate in 15 mL of acetone was added. The mixture was gently purged with nitrogen and allowed to react for 1 hour at 50 °C. 15 mL of a solution of *N*-methyl-2,2,6,6-tetramethyl-4-aminopiperidine in acetone was then added to the prepolymer and the reaction was continued for 3 hours at 50 °C. Evaporation of the solvent gave a homogeneous, transparent and flexible film.

### Example 16

The antioxidant activity against hydrogen peroxide was determined for the IPN sample of Example 15. The measured values were compared with the antioxidant activity of other types of polymer networks having the same antioxidant and antimicrobial component content, namely with a single polyurethane network sample (containing both the antioxidant and the antimicrobial component in one network) and with a film formed by casting a solution of a mixture of two polyurethane networks, one containing the antioxidant component, and the other containing the antimicrobial component. The samples were tested for antioxidant activity against hydrogen peroxide and its oxidative degradation products produced in the presence of tungstate as a catalyst. The experiment itself was performed according to the following procedure. Eight Eppendorf tubes with the pre-determined amounts of the sample were placed in a thermoblock pre-heated to 37 °C. To each tube 900 µL of buffer, 200 µL of Na₂WO₄.2 H₂O solution in buffer (7.3 mM), 10 µL of fluorescein solution (2.4x10⁻⁵ M) were added, and the polymer was left to swell in this mixture for 50 minutes. Then, 10 µL of hydrogen peroxide solution was added. Throughout the reaction, the tubes were shaken and tempered to 37 °C. The tubes were opened at predetermined time intervals, and the fluorescence of the clear solution was determined using a fluorimeter.
For each sample, the measurement was repeated twice. The results of the experiment are summarized in Fig. 6, which shows the dependence of the scavenging activity of the tested sample on its concentration in the reaction mixture. The results show that the IPN according to Example 15 exhibits a higher scavenging activity against the oxidative action of hydrogen peroxide and its decomposition products than a simple polyurethane network or than a mixture of two polyurethane networks.

### Example 17

The antibacterial activity of the IPN sample from Example 15 was also compared with the performance of the single polyurethane network and with the mixture of two polyurethane networks having the same antimicrobial and antioxidant component content as the IPN. Samples of individual films were tested against *Staphylococcus aureus* and *Pseudomonas aeruginosa* as significant contaminants of chronic wounds. Bacterial cultures were sterile dispersed in a liquid medium and 100 µL of this suspension was evenly inoculated on a 2 cm diameter blood agar wheel. The wheel was immediately covered by the tested film. After a 24 hour incubation at 37 °C, the film was removed from the agar surface and the colony forming units (cfu) counting method on solid medium was subsequently used to determine the film efficacy against individual bacterial strains. From the results shown in the Table 3, it is clear that the highest efficacy against the test strains was found for the IPN according to Example 15.

**Table 3: Antibacterial effect of test films against Staphylococcus aureus and Pseudomonas aeruginosa after 24 hours of incubation.**

| Polymer network type | Microbial strain | Antimicrobial effect (%) |
|---|---|---|
| single polyurethane network | *Staphylococcus aureus* (CCM 4516) | 96.2 |
| mixture of two polyurethane networks | | 97.0 |
| IPN | | 99.8 |
| single polyurethane network | *Pseudomonas aeruginosa* (CCM 1961) | 96.4 |
| mixture of two polyurethane networks | | 97.5 |
| IPN | | 99.7 |

### Industrial applicability

Antimicrobials, antioxidants and other starting materials for the product are commercially available materials. The proposed processes for the preparation of interpenetrating networks employ industry-wide methods of radical copolymerization and stepwise polymerization, which significantly simplifies the manufacture of the preparation on an industrial scale, thereby enhancing the practicability of the invention. The polymer-based preparation for wound healing and wound environment adjustment can be used for therapeutic purposes in the treatment of wounds, such as chronic infected wounds, e.g. venous ulcers, bed sores, burns, where it provides an optimal healing process and easy and painless monitoring of the healing process. The preparation may also be used for the prevention and treatment of antimicrobially contaminated mucosa and fungal diseases of skin and nails. The polymeric preparation of the present invention may also be suitable for procedures in crisis situations requiring quick first aid to injured persons. Applications in veterinary medicine are also available.

## Claims

1. Polymer-based preparation for wound healing, **characterized in that** it contains at least two interpenetrated polymer networks, wherein at least one polymer network contains an antimicrobial component covalently bound within the network structure, and at least one other polymer network contains an antiinflammatory and/or antioxidant component covalently bound within the network structure,
- wherein the antimicrobial component is at least one structural unit A-1 derived from monomer of general formula 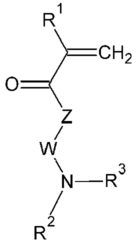
wherein R¹ is -H or -CH₃, Z is -O- or -NH-, W is -(CH₂)ₘ-, wherein m=1 to 12, R² and R³ are independently -H or C₁-C₁₂ alkyl;
wherein the polymer network containing the antimicrobial component A-1 further contains at least one type of structural units selected from group of units derived from acrylamide, methacrylamide, hydroxy alkyl acrylates, hydroxyalkyl methacrylates, hydroxy alkyl acrylamides, hydroxyalkyl methacrylamides, hydroxy-terminated (polyoxyethylene) acrylates, hydroxy-terminated (polyoxyethylene) methacrylates, alkyloxy terminated (polyoxyethylene) acrylates, alkyloxy terminated (polyoxyethylene) methacrylates, hydroxy terminated (polyoxyethylene) acrylamides, hydroxy terminated (polyoxyethylene) methacrylamides, alkyloxy terminated (polyoxyethylene) acrylamides, alkyloxy terminated (polyoxyethylene) methacrylamides;
wherein the polymer network containing the antimicrobial component A-1 further contains at least one type of structural units selected from group of units derived from crosslinking monomers selected from the group consisting of ethylene glycol di(meth)acrylate, oligo (ethylene glycol) di(meth)acrylates, *N,N'*-methylene bis(meth)acrylamide, *N,N'*-ethylenebis(meth)acrylamide, 2,3-dihydroxybutane-1,4-diyl-di(meth)acrylate,.
and/or wherein the antimicrobial component is at least one structural unit A-2 derived from monomer of general formula 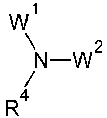 wherein R⁴ is C₁-C₁₂ alkyl, W¹ and W² are independently -(CH₂)ₙ-X¹ or -CH₂-CH₂-(OCH₂-CH₂)ₙ-X², wherein n=1 to 20, X¹ and X² are independently selected from -H, -OH, -NH₂, -NCO, wherein at least one of X¹, X² is selected from -OH, -NH₂, -NCO;
wherein the polymer network containing the antimicrobial component A-2 further contains at least one type of structural units selected from the group consisting of:
- structural units derived from propane-1,2-diol, 2,2-dimethylpropane-1,3-diol, cyclohexane-1,4-diol, 1,4-bis(hydroxymethyl)cyclohexane, 2-(2-hydroxyethoxy)ethane-1-ol, 1,3-diaminopentane, 2,2,4-trimethylhexane-1,6-diisocyanate, 2,4,4-trimethylhexane-1,6-diisocyanate, isophorone diisocyanate, cyclohexane-1,4-diisocyanate, dicyclohexylmethane-4,4'-diisocyanate;
- and/or structural units derived from monomers of general formula: 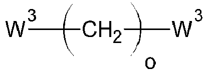 wherein o=3 to 12 and W³ is independently at each occurence -OH, -NH₂ or -NCO;
- and/or structural units derived from monomers of general formula: 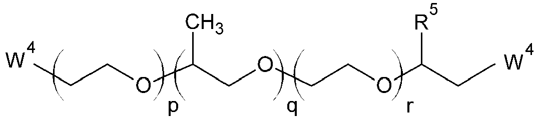 wherein p, q, r are independly an integer from 0 to 100, W⁴ is independently at each occurence -OH, -NH₂ or -NCO, and R⁵ is -H or -CH₃;
wherein the polymer network containing the antimicrobial component A-2 further contains at least one type of structural units selected from low-molecular-weight propane-1,2,3-triol, 2,2-bis(hydroxymethyl)propane-1,3-diol, bis(2-hydroxyethyl)amine, tris(2-hydroxyethyl)amine, 2-(hydroxymethyl)-2-ethylpropane-1,3-diol, and -OH, -NH₂ or -NCO-terminated polymeric precursors based on block and star-like copolymers of ethylene glycol and propylene glycol having a functionality greater than 2.
and
- wherein the antiinflammatory and/or antioxidant component is at least one structural unit B-1 derived from monomers of general formula: 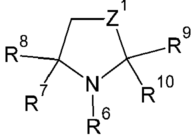
wherein R⁶ is selected from -H, -OH, oxygen radical and C₁-C₄ alkyl, R⁷ to R¹⁰ are independently C₁-C₄ alkyl, Z¹ is -CH(Y¹)- or -CH(Y¹)CH₂-, wherein Y¹ is radical-polymerizable chemical moiety 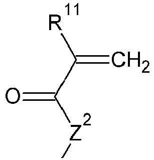
wherein R¹¹ is -H or -CH₃ and Z² is -O- or -NH-;
wherein the polymer network containing the antiinflammatory and/or antioxidant component B-1 further contains at least one type of structural units selected from the group consisting of structural units derived from acrylamide, methacrylamide, hydroxyalkyl acrylates, hydroxyalkyl methacrylates, hydroxyalkyl acrylamides, hydroxyalkyl methacrylamides, hydroxy-terminated (polyoxyethylene) acrylates, hydroxy-terminated (polyoxyethylene) methacrylates, alkyloxy terminated (polyoxyethylene) acrylates, alkyloxy terminated (polyoxyethylene) methacrylates, hydroxy terminated (polyoxyethylene) acrylamides, hydroxy terminated (polyoxyethylene) methacrylamides, alkyloxy terminated (polyoxyethylene) acrylamides, alkyloxy terminated (polyoxyethylene) methacrylamides,
wherein the polymer network containing the antimicrobial component B-1 further contains at least one type of structural units selected from group of units derived from crosslinking monomers selected from the group consisting of ethylene glycol di(meth)acrylate, oligo (ethylene glycol) di(meth)acrylates, *N,N'*-methylene bis(meth)acrylamide, *N,N'*-ethylenebis(meth)acrylamide, 2,3-dihydroxybutane-1,4-diyl-di(meth)acrylate,
and/or at least one structural unit B-2 derived from monomers of general formula: 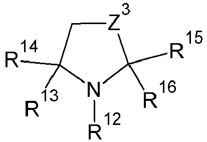 wherein R¹² is selected from -H, -OH, oxygen radical and C₁-C₄ alkyl, R¹³ to R¹⁶ are independently C₁-C₄ alkyl, Z³ is -CH(Y²)- or -CH(Y²)CH₂-, wherein Y² is -OH, -NH₂, -NHC(=O)NH₂, -(CH₂)ₛ-W⁵, wherein s=1 to 6 and W⁵ is -OH or -NH₂;
wherein the polymer network containing the antiinflammatory and/or antioxidant component B-2 further contains at least one type of structural units selected from the group comprising - structural units derived from propane-1,2-diol, 2,2-dimethylpropane-1,3-diol, cyclohexane-1,4-diol, 1,4-bis(hydroxymethyl)cyclohexane, 2-(2-hydroxyethoxy)ethane-1-ol, 1,3-diaminopentane, 2,2,4-trimethylhexane-1,6-diisocyanate, 2,4,4-trimethylhexane-1,6-diisocyanate, isophorone diisocyanate, cyclohexane-1,4-diisocyanate, dicyclohexylmethane-4,4'-diisocyanate;
- and/or structural units derived from monomers of general formula: 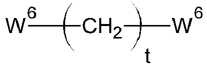 wherein t=3 to 12, and W⁶ is independently at each occurence -OH, -NH₂ or -NCO;
- and/or structural units derived from monomers of general formula: 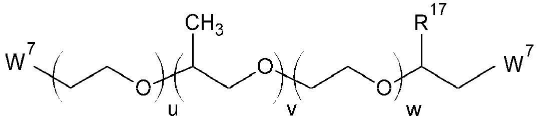 wherein u, v, w are independly an integer from 0 to 100, R¹⁷ is -H or -CH₃ and W⁷ is independently at each occurence -OH, -NH₂ or -NCO;
wherein the polymer network containing the antimicrobial component B-2 further contains at least one type of structural units selected from propane-1,2,3-triol, 2,2-bis(hydroxymethyl)propane-1,3-diol, bis(2-hydroxyethyl)amine, tris(2-hydroxyethyl)amine, 2-(hydroxymethyl)-2-ethylpropane-1,3-diol, and -OH, -NH₂ or -NCO-terminated polymeric precursors based on block and star-like copolymers of ethylene glycol and propylene glycol having a functionality greater than 2.

2. The preparation according to claim 1, wherein the structural units A-1 are derived from *N*-[3-(dimethylamino)propyl]methacrylamide or [2-(dimethylamino)ethyl]methacrylate,
and/or the structural units A-2 are derived from *N,N*-bis(2-hydroxyethyl)methylamine.

3. The preparation according to any one of claims 1 to 2, wherein the content of the structural units A-1 in the polymer network is 1 to 99 mol%, preferably 2 to 30 mol%,
and/or the content of the structural units A-2 in the polymer network is 1 to 99 mol%, preferably 1 to 50 mol%.

4. The preparation according to claim 1, wherein the structural units B-1 are derived from *N*-(2,2,6,6-tetramethylazinan-4-yl) methacrylamide or 2,2,6,6-tetramethylazinan-4-yl methacrylate, and/or the structural units B-2 are derived from 4-amino-2,2,6,6-tetramethylpiperidine, 4-amino-2,2,6,6-tetramethylpiperidine-1-oxyl, 4-hydroxy-2,2,6,6-tetramethyl-piperidine or 4-hydroxy-2,2,6,6-tetramethyl-piperidine-1-oxyl.

5. The preparation according to any one of claims 1 or 4, wherein the content of B-1 structural units in the polymer network is 1 to 45 mol%, preferably 1.5 to 15 mol%, and/or the content of the structural units B-2 in the polymer network is 0.1 to 20 % by weight.

6. The preparation according to any one of the preceding claims, wherein the weight ratio of the polymer network containing the antimicrobial component: the polymer network containing the antiinflammatory and/or antioxidant component is in the range of 0.1 to 10.

7. A method of preparation of the preparation according to any one of the preceding claims, wherein the first polymer network is prepared by polymerization of first monomers, the said first polymer network is subsequently impregnated with second monomers, and the second monomers are then polymerized to form a second polymer network; wherein the polymerizations are independently selected from the group comprising radical polymerizations, and polyadditions;
- the polymer network containing antimicrobial structural units A-1 is prepared by radical polymerization,
- the polymer network containing antimicrobial structural units A-2 is prepared by polyaddition,
- the polymer network containing antiinflammatory and/or antioxidant structural units B-1 is prepared by radical polymerization,
- the polymer network containing antiinflammatory and/or antioxidant structural units B-2 is prepared by polyaddition.

8. A pharmaceutical composition, containing the polymer-based preparation according to any one of claims 1 to 6, and at least one pharmaceutical excipient selected from the group comprising stabilizers, emulsifiers or viscosity enhancers, as well as paraffins, vegetable oils, animal fats, synthetic acylglycerols, waxes, polyalkylsiloxanes, colloidal silicon dioxide with fatty oils, starch, cellulose derivatives, carbomers, magnesium-aluminium silicates, gelatine, surfactants, lubricants, substances with adsorbent properties, water, glycerol, active charcoal; preferably the pharmaceutical composition is in the form selected from suspension, emulsion, ointment, cream, gel, paste, suppositories, globules, tablets, multi-layer wound dressing, wound dressing films.

9. Polymer-based preparation according to any one of claims 1 to 6 for use in wound healing, in particular for use in inhibiting microbial infections and/or inflammatory processes in the wound.

10. Polymer-based preparation according to any one of claims 1 to 6 for use in treatment of diseases of skin, nails and mucous tissues, in particular diseases caused by microbial infection and/or by inflammatory process.

## Patentansprüche

1. Polymerbasiertes Präparat zur Wundheilung, **dadurch gekennzeichnet, dass** es mindestens zwei interpenetrierte Polymernetzwerke enthält, wobei mindestens ein Polymernetzwerk eine innerhalb der Netzwerkstruktur kovalent gebundene antimikrobielle Komponente enthält und mindestens ein weiteres Polymernetzwerk eine entzündungshemmende und/oder antioxidative kovalent innerhalb der Netzwerkstruktur gebundene Komponente enthält,
- wobei die antimikrobielle Komponente mindestens eine Struktureinheit A-1 ist, abgeleitet von einem Monomer der allgemeinen Formel 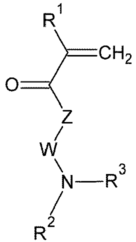
worin R¹ ist -H oder -CH₃, Z ist -O- oder -NH-, W ist -(CH₂)ₘ-, worin m=1 bis 12, R² und R³ sind unabhängig -H oder C₁-C₁₂ Alkyl;
wobei das Polymernetzwerk, das die antimikrobielle Komponente A-1 enthält, weiterhin mindestens einen Typ von Struktureinheiten enthält, ausgewählt aus einer Gruppe von Einheiten abgeleitet von Acrylamid, Methacrylamid, Hydroxyalkylacrylaten, Hydroxyalkylmethacrylaten, Hydroxyalkylacrylamiden, Hydroxyalkylmethacrylamiden, Hydroxy-terminierten (Polyoxyethylen)-Acrylaten, Hydroxy-terminierten (Polyoxyethylen)-Methacrylaten, Alkyloxy-terminierten (Polyoxyethylen)-Acrylaten, Alkyloxy-terminierten (Polyoxyethylen)-Methacrylaten, Hydroxy-terminierten (Polyoxyethylen)-Acrylamiden, Hydroxy-terminierten (Polyoxyethylen)-Methacrylamiden, Alkyloxy-terminierten (Polyoxyethylen)-Acrylamiden, Alkyloxy-terminierten (Polyoxyethylen)-Methacrylamiden;
wobei das Polymernetzwerk, das die antimikrobielle Komponente A-1 enthält, weiterhin mindestens einen Typ von Struktureinheiten enthält, ausgewählt aus einer Gruppe von Einheiten abgeleiteten von vernetzenden Monomeren, ausgewählten aus der Gruppe bestehend aus Ethylenglycoldi(meth)acrylat, Oligo(ethylenglycol)di(meth)acrylate, *N,N*'-Methylen-bis(meth)acrylamid, *N,N'*-Ethylenbis(meth)acrylamid, 2,3-Dihydroxybutan-1,4-diyl-di(meth)acrylat,
und/oder wobei die antimikrobielle Komponente mindestens eine Struktureinheit A-1 ist, abgeleitet von einem Monomer der allgemeinen Formel 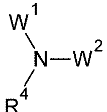 worin R⁴ ist C₁-C₁₂ Alkyl, W¹ und W² sind unabhängig -(CH₂)ₙ-X¹ oder -CH₂-CH₂-(OCH₂-CH₂)ₙ-X², worin n=1 bis 20, X¹ und X² sind unabhängig ausgewählt aus -H, -OH, -NH₂, -NCO, worin mindestens eines von X¹, X² ist ausgewählt aus -OH, -NH₂, -NCO;
wobei das Polymernetzwerk, das die antimikrobielle Komponente A-2 enthält, ferner mindestens einen Typ von Struktureinheiten enthält, ausgewählt aus der Gruppe bestehend aus:
- Struktureinheiten abgeleitet von Propan-1,2-diol, 2,2-Dimethylpropan-1,3-diol, Cyclohexan-1,4-diol, 1,4-Bis(hydroxymethyl)cyclohexan, 2-(2-Hydroxyethoxy)ethan-1-ol, 1,3-Diaminopentan, 2,2,4-Trimethylhexan-1,6-diisocyanat, 2,4,4-Trimethylhexan-1,6-diisocyanat, Isophorondiisocyanat, Cyclohexan-1,4-diisocyanat, Dicyclohexylmethan-4,4'-diisocyanat;
- und/oder Struktureinheiten abgeleitet von Monomeren der allgemeinen Formel: 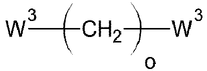 worin o=3 bis 12 und W³ ist bei jedem Auftreten unabhängig -OH, -NH₂ oder -NCO;
- und/oder Struktureinheiten abgeleitet von Monomeren der allgemeinen Formel: 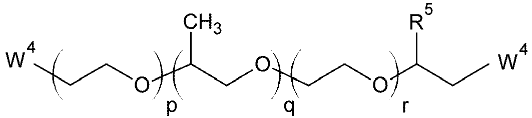 worin p, q, r sind unabhängig eine ganze Zahl von 0 bis 100, W⁴ ist bei jedem Auftreten unabhängig -OH, -NH₂ oder -NCO, und R⁵ ist -H oder -CH₃;
wobei das Polymernetzwerk, das die antimikrobielle Komponente A-2 enthält, weiterhin mindestens einen Typ von Struktureinheiten enthält, ausgewählt aus Propan-1,2,3-triol mit niedrigem Molekulargewicht, 2,2-Bis(hydroxymethyl)propan-1,3-diol, Bis(2-hydroxyethyl)amin, Tris(2-hydroxyethyl)amin, 2-(Hydroxymethyl)-2-ethylpropan-1,3-diol und-OH, -NH₂ oder -NCO-terminierte polymere Vorstufen basierend auf block- und sternförmige Copolymere von Ethylenglykol und Propylenglykol mit einer Funktionalität von mehr als 2;
und
- wobei die entzündungshemmende und/oder antioxidative Komponente mindestens eine Struktureinheit B-1 ist, abgeleitet von Monomeren der allgemeinen Formel: 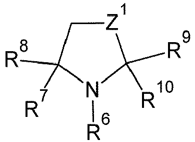
worin R⁶ ist ausgewählt aus -H, -OH, Sauerstoffradikal und C₁-C₄Alkyl, R⁷ bis R¹⁰ sind unabhängig C₁-C₄ Alkyl, Z¹ ist -CH(Y¹)- oder -CH(Y¹)CH₂-, worin Y¹ ist eine radikalisch polymerisierbare chemische Einheit 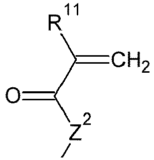
worin R¹¹ ist -H oder -CH₃ und Z² ist -O- oder -NH-;
wobei das Polymernetzwerk, das die entzündungshemmende und/oder antioxidative Komponente B-1 enthält, weiterhin mindestens einen Typ von Struktureinheiten enthält, ausgewählt aus einer Gruppe von Einheiten abgeleitet von Acrylamid, Methacrylamid, Hydroxyalkylacrylaten, Hydroxyalkylmethacrylaten, Hydroxyalkylacrylamiden, Hydroxyalkylmethacrylamiden, Hydroxy-terminierten (Polyoxyethylen)-Acrylaten, Hydroxy-terminierten (Polyoxyethylen)-Methacrylaten, Alkyloxy-terminierten (Polyoxyethylen)-Acrylaten, Alkyloxy-terminierten (Polyoxyethylen)-Methacrylaten, Hydroxy-terminierten (Polyoxyethylen)-Acrylamiden, Hydroxy-terminierten (Polyoxyethylen)-Methacrylamiden, Alkyloxy-terminierten (Polyoxyethylen)-Acrylamiden, Alkyloxy-terminierten (Polyoxyethylen)-Methacrylamiden; wobei das Polymernetzwerk, das die entzündungshemmende und/oder antioxidative Komponente B-1 enthält, weiterhin mindestens einen Typ von Struktureinheiten enthält, ausgewählt aus einer Gruppe von Einheiten abgeleiteten von vernetzenden Monomeren, ausgewählten aus der Gruppe bestehend aus Ethylenglycoldi(meth)acrylat, Oligo(ethylenglycol)di(meth)acrylate, *N,N'-*Methylen-bis(meth)acrylamid, *N,N'*-Ethylenbis(meth)acrylamid, 2,3-Dihydroxybutan-1,4-diyl-di(meth)acrylat,
und/oder mindestens eine Struktureinheit B-2 ist, abgeleitet von Monomeren der allgemeinen Formel: 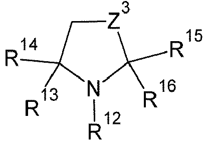 worin R¹² ist ausgewählt aus -H, -OH, Sauerstoffradikal und C₁-C₄ Alkyl, R¹³ bis R¹⁶ sind unabhängig C₁-C₄ Alkyl, Z³ ist -CH(Y²)- oder -CH(Y²)CH₂-, worin Y² ist -OH, -NH₂,-NHC(=O)NH₂, -(CH₂)ₛ-W⁵, worin s=1 bis 6 and W⁵ ist -OH oder -NH₂;
wobei das Polymernetzwerk, das die entzündungshemmende und/oder antioxidative Komponente B-2 enthält, ferner mindestens einen Typ von Struktureinheiten enthält, ausgewählt aus der Gruppe bestehend aus:
- Struktureinheiten abgeleitet von Propan-1,2-diol, 2,2-Dimethylpropan-1,3-diol, Cyclohexan-1,4-diol, 1,4-Bis(hydroxymethyl)cyclohexan, 2-(2-Hydroxyethoxy)ethan-1-ol, 1,3-Diaminopentan, 2,2,4-Trimethylhexan-1,6-diisocyanat, 2,4,4-Trimethylhexan-1,6-diisocyanat, Isophorondiisocyanat, Cyclohexan-1,4-diisocyanat, Dicyclohexylmethan-4,4'-diisocyanat;
- und/oder Struktureinheiten abgeleitet von Monomeren der allgemeinen Formel: 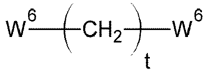 worin t=3 bis 12, und W⁶ ist bei jedem Auftreten unabhängig -OH, -NH₂ oder -NCO;
- und/oder Struktureinheiten abgeleitet von Monomeren der allgemeinen Formel: 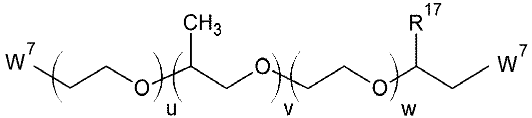 wherein u, v, w sind unabhängig eine ganze Zahl von 0 bis 100, R¹⁷ ist -H oder -CH₃ und W⁷ ist bei jedem Auftreten unabhängig -OH, -NH₂ oder -NCO;
wobei das Polymernetzwerk, das die entzündungshemmende und/oder antioxidative Komponente B-2 enthält, weiterhin mindestens einen Typ von Struktureinheiten enthält, ausgewählt aus Propan-1,2,3-triol, 2,2-Bis(hydroxymethyl)propan-1,3-diol, Bis(2-hydroxyethyl)amin, Tris(2-hydroxyethyl)amin, 2-(Hydroxymethyl)-2-ethylpropan-1,3-diol und -OH, -NH₂ oder -NCO-terminierte polymere Vorstufen basierend auf block- und sternförmige Copolymere von Ethylenglykol und Propylenglykol mit einer Funktionalität von mehr als 2.

2. Präparat nach Anspruch 1, wobei die Struktureinheiten A-1 von *N-*[3-(Dimethylamino)propyl]methacrylamid oder [2-(Dimethylamino)ethyl]methacrylat abgeleitet sind. und/oder die Struktureinheiten A-2 abgeleitet sind von *N,N*-Bis(2-hydroxyethyl)methylamin.

3. Präparat nach einem der Ansprüche 1 bis 2, wobei der Gehalt der Struktureinheiten A-1 im Polymernetzwerk 1 bis 99 Mol-%, vorzugsweise 2 bis 30 Mol-% beträgt;
und/oder der Gehalt der Struktureinheiten A-2 im Polymernetzwerk 1 bis 99 Mol-%, vorzugsweise 1 bis 50 Mol-% beträgt.

4. Präparat nach Anspruch 1, wobei die Struktureinheiten B-1 abgeleitet sind von *N*-(2,2,6,6-Tetramethylazinan-4-yl)methacrylamid oder 2,2,6,6-Tetramethylazinan-4- ylmethacrylat, und/oder die Struktureinheiten B-2 sind abgeleitet von 4-Amino-2,2,6,6-tetramethylpiperidin, 4-Amino-2,2,6,6-tetramethylpiperidin-1-oxyl, 4-Hydroxy-2,2,6,6-tetramethyl-piperidin oder 4-Hydroxy-2,2,6,6-tetramethyl-piperidin-1-oxyl.

5. Präparat nach einem der Ansprüche 1 oder 4, wobei der Gehalt der Struktureinheiten B-1 im Polymernetzwerk 1 bis 45 Mol-%, vorzugsweise 1,5 bis 15 Mol-% beträgt, und/oder der Gehalt der Struktureinheiten B-2 im Polymernetzwerk 0,1 bis 20 Gew.-% beträgt.

6. Präparat nach einem der vorhergehenden Ansprüche, wobei das Gewichtsverhältnis des Polymernetzwerks, das die antimikrobielle Komponente enthält : des Polymernetzwerks, das die entzündungshemmende und/oder antioxidative Komponente enthält, im Bereich von 0,1 bis 10 liegt.

7. Verfahren zur Herstellung des Präparats nach einem der vorhergehenden Ansprüche, wobei das erste Polymernetzwerk durch Polymerisation von ersten Monomeren hergestellt wird, das erste Polymernetzwerk anschließend mit zweiten Monomeren imprägniert wird, und die zweiten Monomere dann polymerisiert werden um ein zweites Polymernetzwerk zu bilden; wobei die Polymerisationen unabhängig ausgewählt sind aus der Gruppe umfassend radikalische Polymerisationen und Polyadditionen;
- das antimikrobielle Struktureinheiten A-1 enthaltende Polymernetzwerk durch radikalische Polymerisation hergestellt wird,
- das antimikrobielle Struktureinheiten A-2 enthaltende Polymernetzwerk durch Polyaddition hergestellt wird,
- das Polymernetzwerk mit entzündungshemmenden und/oder antioxidativen Struktureinheiten B-1 durch radikalische Polymerisation hergestellt wird,
- das Polymernetzwerk mit entzündungshemmenden und/oder antioxidativen Struktureinheiten B-2 durch Polyaddition hergestellt wird.

8. Pharmazeutische Zusammensetzung, enthaltend das polymerbasiertes Präparat nach einem der Ansprüche 1 bis 6 und mindestens einen pharmazeutischen Hilfsstoff ausgewählt aus der Gruppe umfassend Stabilisatoren, Emulgatoren oder Viskositätsverstärker, sowie Paraffine, Pflanzenöle, tierische Fette, synthetische Acylglycerine, Wachse, Polyalkylsiloxane, kolloidales Siliciumdioxid mit fetten Ölen, Stärke, Cellulosederivate, Carbomere, Magnesium-Aluminium-Silikate, Gelatine, Tenside, Gleitmittel, Stoffe mit adsorbierenden Eigenschaften, Wasser, Glycerin, Aktivkohle; vorzugsweise ist die pharmazeutische Zusammensetzung in der Form ausgewählt aus Suspension, Emulsion, Salbe, Creme, Gel, Paste, Zäpfchen, Globuli, Tabletten, mehrschichtigem Wundverband, Wundverbandsfilmen.

9. Polymerbasiertes Präparat nach einem der Ansprüche 1 bis 6 zur Verwendung bei der Wundheilung, insbesondere zur Verwendung bei der Hemmung mikrobieller Infektionen und/oder entzündlicher Prozesse in der Wunde.

10. Polymerbasiertes Präparat nach einem der Ansprüche 1 bis 6 zur Verwendung bei der Behandlung von Erkrankungen der Haut, Nägel und Schleimhäute, insbesondere Erkrankungen, die durch mikrobielle Infektionen und/oder durch entzündliche Prozesse verursacht werden.

## Revendications

1. Préparation à base de polymère pour la cicatrisation des plaies, **caractérisée en ce qu'**elle contient au moins deux réseaux polymères interpénétrés, où au moins un réseau polymère contient un composant antimicrobien lié de manière covalente dans la structure du réseau, et au moins un autre réseau polymère contient un anti-inflammatoire et/ou composant antioxydant lié de manière covalente dans la structure du réseau,
- où le composant antimicrobien est au moins une unité structurale A-1 dérivée d'un monomère de formule générale 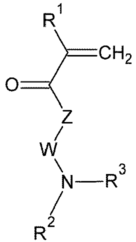
où R¹ est -H ou -CH₃, Z est -O- ou -NH-, W est -(CH₂)ₘ-, où m=1 à 12, R² et R³ sont indépendamment -H ou alkyle en C₁-C₁₂;
où le réseau polymère contenant le composant antimicrobien A-1 contient en outre au moins un type d'unités structurelles choisies dans le groupe d'unités dérivées de l'acrylamide, du méthacrylamide, des hydroxyalkylacrylates, des hydroxyalkyleméthacrylates, des hydroxyalkylacrylamides, des hydroxyalkyleméthacrylamides, des (polyoxyéthylène) acrylates à terminaison hydroxy, des (polyoxyéthylène) méthacrylates à terminaison hydroxy, des (polyoxyéthylène) acrylates à terminaison alkyloxy, des (polyoxyéthylène) méthacrylates à terminaison alkyloxy, des (polyoxyéthylène) acrylamides à terminaison hydroxy, des (polyoxyéthylène) méthacrylamides à terminaison hydroxy, des (polyoxyéthylène) acrylamides à terminaison alkyloxy, des (polyoxyéthylène) méthacrylamides à terminaison alkyloxy;
où le réseau polymère contenant le composant antimicrobien A-1 contient en outre au moins un type d'unités structurelles choisies dans le groupe d'unités dérivées de monomères de réticulation choisis dans le groupe consistant en di(méth)acrylate d'éthylèneglycol, di(méth)acrylates d'oligo(éthylèneglycol), N,N'-méthylène bis(méth)acrylamide, N,N'-éthylènebis(méth)acrylamide, 2,3-dihydroxybutane-1,4-diyl-di(méth)acrylate,
et/ou le composant antimicrobien est au moins une unité structurale A-2 dérivée d'un monomère de formule générale 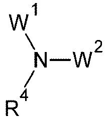 où R⁴ est C₁-C₁₂ alkyle en C₁-C₁₂, W¹ et W² sont indépendamment -(CH₂)ₙ-X¹ ou -CH₂-CH₂-(OCH₂-CH₂)ₙ-X², où n=1 à 20, X¹ et X² sont indépendamment choisi parmi -H, -OH, -NH₂, -NCO, où au moins un de X¹, X² est choisi parmi -OH, -NH₂, -NCO;
où le réseau polymère contenant le composant antimicrobien A-2 contient en outre au moins un type d'unités structurelles choisies dans le groupe consistant en:
- unités structurelles dérivées du propane-1,2-diol, du 2,2-diméthylpropane-1,3-diol, du cyclohexane-1,4-diol, du 1,4-bis(hydroxyméthyl)cyclohexane, du 2-(2-hydroxyéthoxy)éthane
- 1-ol, 1,3-diaminopentane, 2,2,4-triméthylhexane-1,6-diisocyanate, 2,4,4-triméthylhexane-1,6-diisocyanate, isophorone diisocyanate, cyclohexane-1,4-diisocyanate, dicyclohexylméthane-4,4'-diisocyanate;
- et/ou unités structurelles dérivés de monomères de formule générale: 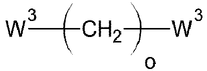 où o=3 à 12 et W³ est indépendamment à chaque occurrence -OH, -NH₂ ou -NCO;
- et/ou unités structurelles dérivés de monomères de formule générale: 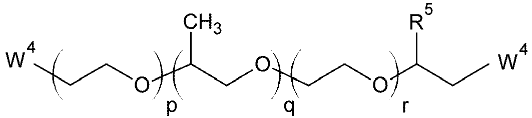 où p, q, r sont indépendamment un nombre entier de 0 à 100, W⁴ est indépendamment à chaque occurrence -OH, -NH₂ ou -NCO, et R⁵ est -H ou -CH₃;
où le réseau polymère contenant le composant antimicrobien A-2 contient en outre au moins un type d'unités structurelles choisies parmi le propane-1,2,3-triol de bas poids moléculaire, le 2,2-bis(hydroxyméthyl)propane-1,3-diol, bis(2-hydroxyéthyl)amine, tris(2-hydroxyéthyl)amine, 2-(hydroxyméthyl)-2-éthylpropane-1,3-diol et précurseurs polymères à terminaison -OH, -NH₂ ou -NCO à base de copolymères séquencés et étoilés d'éthylène glycol et de propylène glycol ayant une fonctionnalité supérieure à 2;
et
- où le composant anti-inflammatoire et/ou antioxydant est au moins une unité structurelle B-1 dérivée de monomères de formule générale: 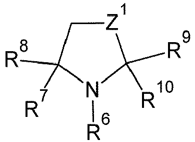
où R⁶ est choisi parmi from -H, -OH, radical oxygène et alkyle en C₁-C₄, R⁷ à R¹⁰ sont indépendamment alkyles en C₁-C₄, Z¹ est -CH(Y¹)- ou -CH(Y¹)CH₂-, où Y¹ est un fragment chimique polymérisable par radicaux 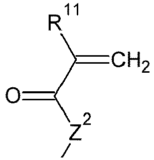
où R¹¹ est -H ou -CH₃ et Z² est -O- ou -NH-;
où le réseau polymère contenant le composant anti-inflammatoire et/ou antioxydant B-1 contient en outre au moins un type d'unités structurelles choisies dans le groupe d'unités structurelles dérivées de l'acrylamide, du méthacrylamide, des hydroxyalkylacrylates, des hydroxyalkyleméthacrylates, des hydroxyalkylacrylamides, des hydroxyalkyleméthacrylamides, des (polyoxyéthylène) acrylates à terminaison hydroxy, des (polyoxyéthylène) méthacrylates à terminaison hydroxy, des (polyoxyéthylène) acrylates à terminaison alkyloxy, des (polyoxyéthylène) méthacrylates à terminaison alkyloxy, des (polyoxyéthylène) acrylamides à terminaison hydroxy, des (polyoxyéthylène) méthacrylamides à terminaison hydroxy, des (polyoxyéthylène) acrylamides à terminaison alkyloxy, des (polyoxyéthylène) méthacrylamides à terminaison alkyloxy;
où le réseau polymère contenant le composant anti-inflammatoire et/ou antioxydant B-1 contient en outre au moins un type d'unités structurelles choisies dans le groupe d'unités dérivées de monomères de réticulation choisis dans le groupe consistant en di(méth)acrylate d'éthylèneglycol, di(méth)acrylates d'oligo(éthylèneglycol), N,N'-méthylène bis(méth)acrylamide, N,N'-éthylènebis(méth)acrylamide, 2,3-dihydroxybutane-1,4-diyl-di(méth)acrylate,
et/ou au moins une unité structurelle B-2 dérivée de monomères de formule générale: 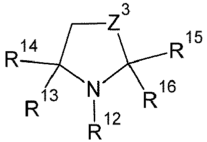 où R¹² est choisi parmi -H, -OH, radical oxygène et alkyle en C₁-C₄, R¹³ à R¹⁶ sont indépendamment alkyles en C₁-C₄, Z³ est -CH(Y²)- ou -CH(Y²)CH₂-, où Y² est -OH, -NH₂, -NHC(=O)NH₂,-(CH₂)ₛ-W⁵, où s=1 à 6 et W⁵ est -OH ou -NH₂;
où le réseau polymère contenant le composant anti-inflammatoire et/ou antioxydant B-2 contient en outre au moins un type d'unités structurelles choisies dans le groupe comprenant
- unités structurales dérivées du propane-1,2-diol, du 2,2-diméthylpropane-1,3-diol, du cyclohexane-1,4-diol, du 1,4-bis(hydroxyméthyl)cyclohexane, du 2-(2-hydroxyéthoxy)éthane-1-ol, 1,3-diaminopentane, 2,2,4-triméthylhexane-1,6-diisocyanate, 2,4,4-triméthylhexane-1,6-diisocyanate, isophorone diisocyanate, cyclohexane-1,4-diisocyanate, dicyclohexylméthane-4,4'-diisocyanate;
- et/ou unités structurelles dérivés de monomères de formule générale: 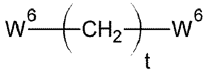 où t=3 à 12, et W⁶ est indépendamment à chaque occurrence -OH, -NH₂ ou -NCO;
- et/ou unités structurelles dérivés de monomères de formule générale: 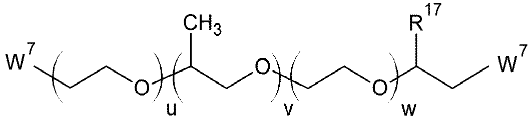 où u, v, w sont indépendamment un nombre entier de 0 à 100, R¹⁷ is -H or -CH₃ and W⁷ est indépendamment à chaque occurrence -OH, -NH₂ ou -NCO;
où le réseau polymère contenant le composant anti-inflammatoire et/ou antioxydant B-2 contient en outre au moins un type d'unités structurelles choisies parmi le propane-1,2,3-triol, le 2,2-bis(hydroxyméthyl)propane-1,3-diol, bis(2-hydroxyéthyl)amine, tris(2-hydroxyéthyl)amine, 2-(hydroxyméthyl)-2-éthylpropane-1,3-diol et précurseurs polymères à terminaison -OH, -NH₂ ou -NCO à base de copolymères séquencés et étoilés d'éthylène glycol et de propylène glycol ayant une fonctionnalité supérieure à 2

2. Préparation selon la revendication 1, où les unités structurelles A-1 sont dérivées du *N-*[3-(diméthylamino)propyl]méthacrylamide ou du [2-(diméthylamino)éthyl]méthacrylate,
et/ou les unités structurelles A-2 sont dérivées de la *N,N*-bis(2-hydroxyéthyl)méthylamine.

3. Préparation selon l'une quelconque des revendications 1 à 2, où la teneur des unités structurelles A-1 dans le réseau polymère est de 1 à 99 % en moles, de préférence de 2 à 30 % en moles,
et/ou la teneur des unités structurelles A-2 dans le réseau polymère est de 1 à 99 % en moles, de préférence de 1 à 50 % en moles.

4. Préparation selon la revendication 1, où les unités structurelles B-1 sont dérivées du *N*-(2,2,6,6-tétraméthylazinan-4-yl)méthacrylamide ou du 2,2,6,6-tétraméthylazinan-4-ylméthacrylate, et/ou les unités structurelles B-2 sont dérivées de 4-amino-2,2,6,6-tétraméthylpipéridine, 4-amino-2,2,6,6-tétraméthylpipéridine-1-oxyle, 4-hydroxy-2,2,6,6-tétraméthyl-pipéridine ou 4-hydroxy-2,2,6,6-tétraméthylpipéridine-1-oxyle.

5. Préparation selon l'une quelconque des revendications 1 ou 4, où la teneur des unités structurelles B-1 dans le réseau polymère est de 1 à 45 % en moles, de préférence de 1,5 à 15 % en moles, et/ou la teneur de unités structurelles B-2 dans le réseau polymère est de 0,1 à 20 % en poids.

6. Préparation selon l'une quelconque des revendications précédentes, où le rapport pondéral du réseau polymère contenant le composant antimicrobien : le réseau polymère contenant le composant anti-inflammatoire et/ou antioxydant est compris entre 0,1 et 10.

7. Procédé de préparation de la préparation selon l'une quelconque des revendications précédentes, où le premier réseau polymère est préparé par polymérisation de premiers monomères, ledit premier réseau polymère est ensuite imprégné de deuxièmes monomères, et les deuxièmes monomères sont ensuite polymérisés pour former un deuxième réseau polymère; où les polymérisations sont indépendamment choisies dans le groupe comprenant les polymérisations radicalaires et les polyadditions ;
- le réseau polymère contenant des unités structurelles antimicrobiennes A-1 est préparé par polymérisation radicalaire,
- le réseau polymère contenant les unités structurelles antimicrobiennes A-2 est préparé par polyaddition,
- le réseau polymère contenant des unités structurelles anti-inflammatoires et/ou antioxydantes B-1 est préparé par polymérisation radicalaire,
- le réseau polymère contenant des unités structurelles anti-inflammatoires et/ou antioxydantes B-2 est préparé par polyaddition.

8. Composition pharmaceutique, contenant la préparation à base de polymère selon l'une quelconque des revendications 1 à 6, et au moins un excipient pharmaceutique choisi dans le groupe comprenant les stabilisants, les émulsifiants ou les viscosifiants, ainsi que les paraffines, les huiles végétales, les graisses animales. acylglycérols synthétiques, cires, polyalkylsiloxanes, dioxyde de silicium colloïdal avec huiles grasses, amidon, dérivés cellulosiques, carbomères, silicates de magnésium-aluminium, gélatine, tensioactifs, lubrifiants, substances à propriétés adsorbantes, eau, glycérol, charbon actif; de préférence, la composition pharmaceutique est sous forme choisie parmi suspension, émulsion, pommade, crème, gel, pâte, suppositoires, globules, comprimés, pansement multicouche, films de pansement.

9. Préparation à base de polymère selon l'une quelconque des revendications 1 à 6 pour utilisation dans la cicatrisation des plaies, en particulier pour utilisation dans l'inhibition des infections microbiennes et/ou des processus inflammatoires de la plaie.

10. Préparation à base de polymère selon l'une quelconque des revendications 1 à 6 pour utilisation dans le traitement des maladies de la peau, des ongles et des muqueuses, en particulier les maladies provoquées par une infection microbienne et/ou par un processus inflammatoire.
